(19) Europäisches Patentamt
European Patent Office
Office européen des brevets

(11) Veröffentlichungsnummer: **0 332 580 B1**

# (12) EUROPÄISCHE PATENTSCHRIFT

(45) Veröffentlichungstag der Patentschrift: **12.10.94**

(51) Int. Cl.$^{5}$: **C07D 493/22**, A61K 31/365, A23K 1/17, A01N 43/90, //(C07D493/22,313:00,311:00, 311:00,307:00)

(21) Anmeldenummer: **89810161.3**

(22) Anmeldetag: **02.03.89**

Die Akte enthält technische Angaben, die nach dem Eingang der Anmeldung eingereicht wurden und die nicht in dieser Patentschrift enthalten sind.

## (54) Von Milbemycinen ableitbare Insektizide und Parasitizide.

(30) Priorität: **11.03.88 CH 934/88**

(43) Veröffentlichungstag der Anmeldung: **13.09.89 Patentblatt 89/37**

(45) Bekanntmachung des Hinweises auf die Patenterteilung: **12.10.94 Patentblatt 94/41**

(84) Benannte Vertragsstaaten: **AT BE CH DE ES FR GB GR IT LI LU NL SE**

(56) Entgegenhaltungen:
**EP-A- 0 143 747**
**EP-A- 0 189 159**
**EP-A- 0 282 456**
**DE-A- 3 631 387**

(73) Patentinhaber: **CIBA-GEIGY AG**
**Klybeckstrasse 141**
**CH-4002 Basel (CH)**

(72) Erfinder: **O'Sullivan, Anthony Cornelius, Dr.**
**Habsburgerstrasse 38**
**CH-4055 Basel (CH)**

**EP 0 332 580 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, von Milbemycinen ableitbare Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten.

Die neuen Verbindungen haben die allgemeine Formel I

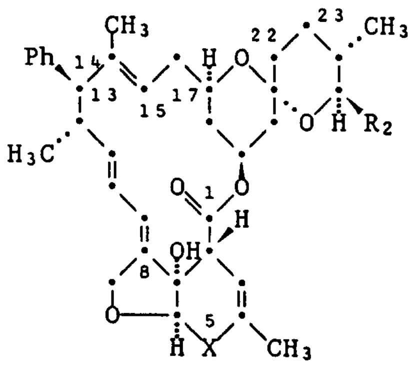

(I)

in welcher

X eine der Gruppen -CH($OR_1$)-, -C(O)- oder -C(=N-OR)- repräsentiert;

$R_1$ Wasserstoff oder eine OH-Schutzgruppe bedeutet;

R für Wasserstoff, eine OH-Schutzgruppe, eine Alkyl- oder Cycloalkylgruppe steht;

$R_2$ für Methyl, Ethyl, Isopropyl oder sek.Butyl steht;

und

Ph einen durch $R_a$, $R_b$, $R_c$ und $R_d$ substituierten Phenylring darstellt,

wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

Unter dem Begriff Alkyl als Substituent oder als Bestandteil eines Substituenten sind je nach Zahl der angegebenen Koblenstoffatome Methyl, Ethyl, Propyl, Butyl, Pentyl, Hexyl, Heptyl, Octyl, Nonyl und Decyl zu verstehen, sowie alle in Betracht kommenden Isomeren davon, wie beispielsweise Isopropyl, Isobutyl, tert.-Butyl und Isopentyl. R als Alkyl steht bevorzugt für Alkyl mit 1 bis 8, insbesondere 1 bis 4, Kohlenstoffatomen.

Cycloalkyl steht bevorzugt für $C_3$-$C_6$-Cycloalkyl, also für Cyclopropyl, Cyclobutyl, Cyclopentyl und Cyclohexyl.

$C_2$-$C_{10}$-Alkoxyalkyl steht für einen Alkylrest, dessen Kohlenstoffgerüst aus bis zu 10 C-Atomen besteht und an einer Stelle durch ein Sauerstoffatom unterbrochen ist. Bevorzugt sind Alkoxyalkylgruppen mit 1 bis 6 C-Atomen, wie beispielsweise $-CH_2-O-CH_3$, $-CH_2-O-C_2H_5$, $-CH_2CH_2-O-C_2H_5$, $-CH_2CH_2-O-CH_3$, $-CH(CH_3)-O-CH_3$, $-CH_2-O-C(CH_3)_3$, $-C(CH_3)_2-O-CH_3$ oder $-CH_2CH_2CH_2-O-nC_3H_7$.

$C_2$-$C_{10}$-Alkenyl steht für einen geradkettigen oder verzweigten, acyclischen, aliphatischen Rest mit einer Doppelbindung. Besonders erwähnenswert ist $C_2$-$C_4$-Alkenyl, beispielsweise Vinyl und Allyl.

Von den $C_1$-$C_{10}$-Alkoxygruppen sind solche mit 1 bis 6 C-Atomen bevorzugt. Beispiele sind Propoxy, Ethoxy und insbesondere Methoxy.

$C_2$-$C_{10}$-Alkoxyalkoxy steht für einen Alkoxyrest, dessen Kohlenstoffgerüst aus bis zu 10 C-Atomen besteht und an einer Stelle durch ein Sauerstoffatom unterbrochen ist. Bevorzugt sind Alkoxyalkoxygruppen mit 2 bis 6 C-Atomen, wie beispielsweise $-O-CH_2CH_2-O-C_2H_5$, $-O-CH_2CH_2-O-CH_3$, $-O-CH_2CH_2CH_2-O-nC_3H_7$, $-O-CH_2-C(CH_3)_2-O-CH_3$, $-O-CH_2-O-CH_3$ oder $-O-CH_2CH_2CH_2CH_2-O-CH_3$, sowie ferner $-O-CH_2CH_2-O-CH_2-C(CH_3)_3$.

Als substituierte Phenyl- und Phenoxyreste $R_a$, $R_b$, $R_c$ und $R_d$ kommen vorzugsweise solche in Betracht, welche durch 1 bis 3 Substituenten der Gruppe $C_1$-$C_3$-Alkyl oder $C_1$-$C_3$-Alkoxy substituiert sind, und insbesondere solche, bei denen die Gesamtzahl der Kohlenstoffatome aller Substituenten eines Ringes zusammengenommen die Zahl 5 nicht übersteigt. Bevorzugte Substituenten dar Phenyl- und Phenoxyreste sind Methyl und Methoxy.

Ph steht insbesondere für Phenyl, einen ein- bis dreifach durch Methyl substituierten Phenylrest, einen ein- bis dreifach durch Methoxy substituierten Phenylrest oder einen durch Phenyl oder Phenoxy monosubstituierten Phenylrest.

Als Strukturelemente, welche durch "Ph" symbolisiert werden, kommen solche in Betracht, die sich von nach üblichen chemischen Methoden herstellbaren Verbindungen ableiten lassen.

Unter OH-Schutzgruppen für die Substituenten R und $R_1$ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste $R_4$-C(O)-, wobei $R_4$ für $C_1$-$C_{10}$-Alkyl, $C_1$-$C_{10}$-Haloalkyl oder eine unsubstituierte oder durch einen oder mehrere Substituenten ausgewählt aus der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierte Gruppe aus der Reihe Phenyl und Benzyl steht und vorzugsweise $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet. Als geeignete Silylgruppen für $R_1$ und R kommt der Rest -Si($R_5$)($R_6$)($R_7$) in Frage, wobei $R_5$, $R_6$ und $R_7$ vorzugsweise unabhängig voneinander für $C_1$-$C_4$-Alkyl, Benzyl oder Phenyl stehen und beispielsweise gemeinsam mit dem Siliciumatom eine der Gruppen Trimethylsilyl, tris(tert.Butyl)silyl, Diphenyl-tert.butylsilyl, bis(Isopropyl)methylsilyl,Triphenylsilyl usw. und insbesondere tert.Butyl-dimethylsilyl bildet. Die 5-OH-Gruppe kann auch verethert als Benzylether oder Methoxyethoxymethylether vorliegen.

Verbindungen der Formel I, worin X die Gruppe -CH($OR_1$)- oder -C( = N-OR)-repräsentiert und $R_1$ und R eine Schutzgruppe darstellen, lassen sich durch einfache, z.B. hydrolytische Abspaltung der Schutzfunktion in die hochaktiven freien 5-Hydroxy-Derivate ($R_1$ = H) oder die 5-Oximderivate (R = H) überführen und haben somit auch Zwischenprodukte-Charakter. Im übrigen wird der biologische Wert dieser Verbindungen durch die Schutzgruppe gar nicht oder nicht in nennenswertem Ausmass gemindert. Die hydrolytische Abspaltung der Schutzgruppe kann mit einer verdünnten Säure, beispielsweise mit 1 %iger p-Toluolsulfonsäure in Methanol oder mit einer wässrigen HF-Lösung in Acetonitril bei -20 °C bis 50 °C, bevorzugt bei 0 °C bis 30 °C, oder auch mit Pyridiniumfluorid in Pyridin erfolgen.

Die vorgenannten Acyl- und Silylgruppen dienen nicht nur als Schutzgruppen für im Substituenten X vorliegende Hydroxygruppen, sondern auch für alle anderen in den erfindungsgemässen Verbindungen oder Vorstufen dieser Verbindungen vorliegenden Hydroxygruppen.

Verbindungen der Formel I, worin X -CH($OR_1$)- und $R_1$ Wasserstoff bedeutet, sind bevorzugt. Acyl- und Silylgruppen als R und $R_1$ sind im allgemeinen als Schutzgruppen aufzufassen.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-A-4,173,571 in Milbemycin-Homologe überführen.

In natürlich vorkommenden Milbemycinen (X = -CH($OR_1$)-, $R_1$ = H; $R_2$ = $CH_3$, $C_2H_5$ oder iso-$C_3H_7$) ist die 13-Position stets lediglich mit Wasserstoff besetzt:

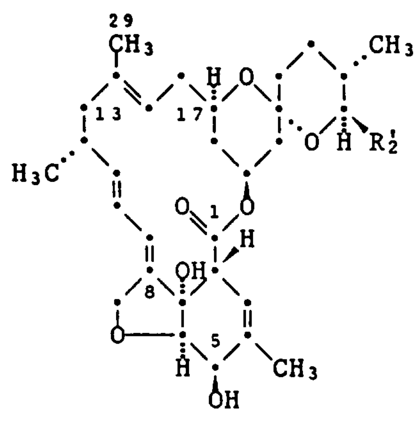

(M)

$R_2'$ = $CH_3$ Milbemycin $A_3$ (US-A-3,950,360)

$R_2'$ = $C_2H_5$ Milbemycin $A_4$ (US-A-3,950,360)

$R_2'$ = iso$C_3H_7$ Milbemycin D (US-A-4,346,171)

$R_2'$ = sec.$C_4H_9$ 13-Deoxy-22,23-dihydro-C-076-Bla-aglycon (US-A-4,173,571, GB-A-1,573,955 und DE-A-2 717 040)

Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen

Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine allylische 13α-Hydroxy-Gruppe besitzen. Die Avermectin-Aglykone sind, wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor.

Folgende Untergruppen von Verbindungen der Formel I gemäss vorliegender Erfindung sind auf Grund ihrer ausgeprägten parasitiziden und insektiziden Wirkung besonders bevorzugt:

Gruppe Ia: Verbindungen der Formel I, worin X -CH($OR_1$)- und $R_1$ Wasserstoff oder eine Schutzgruppe bedeutet, $R_2$ für Methyl, Ethyl, Isopropyl oder sek.-Butyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

Gruppe Ib: Verbindungen der Formel I, worin X -CH($OR_1$)- und $R_1$ Wasserstoff bedeutet, $R_2$ für Methyl, Ethyl, Isopropyl oder sek.-Butyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

Gruppe Ic: Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{20}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl-oder Phenoxyrest stehen.

Gruppe Id: Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy oder $C_2$-$C_{10}$-Alkoxyalkoxy stehen.

Gruppe Ie: Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

Gruppe If: Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_3$-Alkyl, oder $C_1$-$C_3$-Alkoxy, stehen, mit der Massgabe, dass höchstens zwei der Substituenten $R_a$, $R_b$, $R_c$ und $R_d$ eine andere Bedeutung als Wasserstoff haben.

Gruppe Ig: Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, Phenyl oder Phenoxy stehen, mit der Massgabe, dass höchstens zwei der Substituenten $R_a$, $R_b$, $R_c$ und $R_d$ eine andere Bedeutung als Wasserstoff haben.

Gruppe Ih: Verbindungen der Formel I, worin X -CH($OR_1$)- und $R_1$ Wasserstoff bedeutet, $R_2$ für Methyl oder insbesondere für Ethyl steht; und Ph für unsubstituiertes oder durch Methyl, Methoxy, Phenyl oder Phenoxy monosubstituiertes oder durch Methyl oder Methoxy disubstituiertes oder durch Methyl trisubstituiertes Phenyl steht.

Gruppe Ii: Verbindungen der Formel I, worin X -CH($OR_1$)- und $R_1$ Wasserstoff bedeutet, $R_2$ für Methyl oder insbesondere für Ethyl steht; und Ph für unsubstituiertes oder durch Methyl, Methoxy, Phenyl oder Phenoxy monosubstituiertes oder durch Methoxy disubstituiertes Phenyl steht.

Bevorzugte Einzelverbindungen (X = -CH($OR_1$)-, $R_1$ = H) sind:

13β-Phenyl-milbemycin $A_4$,
13β-(2-Methoxyphenyl)-milbemycin $A_4$,
13β-(3-Methoxyphenyl)-milbemycin $A_4$,
13β-(4-Methoxyphenyl)-milbemycin $A_4$,
13β-(2-Methylphenyl)-milbemycin $A_4$,
13β-(4-Biphenylyl)-milbemycin $A_4$,
13β-(4-Phenoxyphenyl)-milbemycin $A_4$ und
13β-(3,4-Dimethoxyphenyl)-milbemycin $A_4$.

Eine ausgeprägte Wirkung zeigen die folgenden Verbindungen:
13β-Phenyl-milbemycin $A_3$
13β-(2-Methylphenyl)-milbemycin $A_4$ und
13β-(4-Methylphenyl)-milbemycin $A_4$.

Die vorliegende Erfindung betrifft neben den Verbindungen der Formel I auch neue Verfahren zu ihrer Herstellung. Es wurde nämlich gefunden, dass man überraschenderweise den Substituenten "Ph" gezielt an das 13-C-Atom des Makrolidmoleküls stereospezifisch in β-Position binden kann, indem man die entsprechende 13β-Halogen-Verbindung oder das entsprechende 15-Halogen-$\Delta^{13,14}$-Derivat mit einer Arylmetall-Verbindung, worin der Arylteil dem Substituenten "Ph" entspricht, in Gegenwart einer katalytischen Menge eines Uebergangsmetallsalzes umsetzt. Die Umsetzung von Verbindungen des

Typs 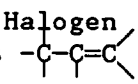

mit Arylmetall-Verbindungen,
katalysiert durch ein Uebergangsmetallsalz, ist aus der Literatur bekannt (Acc. Chem. Res. 1982, 15, 340-348), und diese Umsetzung findet in der Regel unter Inversion statt. Das bedeutet, dass beispielsweise bei der Umsetzung eines entsprechenden 13β-Halogen-milbemycin-Derivats mit einer Arylmetall-Verbindung, katalysiert durch ein Uebergangsmetallsalz, die Entstehung von 13α-Arylmilbemycin-Derivat zu erwarten ist. Ueberraschenderweise erhält man jedoch nur 13β-Arylmilbemycin-Derivate.

Ein weiterer Gegenstand der Erfindung ist somit ein Verfahren zur Herstellung von Verbindungen der Formel I, das darin besteht, dass man eine Verbindung der Formel II

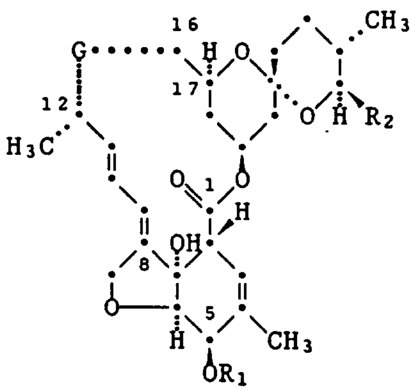

(II)

worin G für eine der Gruppen a oder b

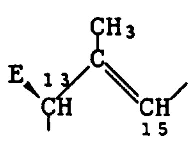 (a) oder 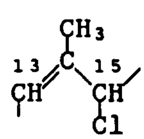 (b)

[= 13β-Halogen-$\Delta^{14,15}$] [= 15-Chlor-$\Delta^{13,14}$]

steht, $R_1$ Wasserstoff oder eine OH-Schutzgruppe bedeutet, $R_2$ die für Formel I angegebenen Bedeutungen hat und E für Chlor, Brom oder Jod steht, mit einer Diarylzink-Verbindung der Formel III

Ph-Zn-Ph (III),

worin Ph die für Formel I angegebene Bedeutung hat, in Gegenwart eines Uebergangsmetallsalzes umsetzt und gewünschtenfalls, wenn $R_1$ für eine OH-Schutzgruppe steht, diese Schutzgruppe hydrolytisch abspaltet.

Im Rahmen der vorliegenden Erfindung sind unter Uebergangsmetallsalzen solche Salze zu verstehen, welche als Metallkomponente ein Uebergangsmetall der Gruppe VIII enthalten. Von den 9 Metallen dieser Gruppe, nämlich Fe, Ru, Os, Co, Rh, Ir, Ni, Pd und Pt werden als Metallkomponente der Uebergangsmetallsalze Co, Ni und Pd bevorzugt. In Betracht kommen rein anorganische Uebergangsmetallsalze wie auch solche, die mit organischen Liganden komplexiert sind. Typische organische Liganden sind Phosphine und organisch substituierte Amine. Charakteristische Vertreter geeigneter Uebergangsmetallsalze sind beispielsweise $NiCl_2$, $CoCl_2$, $[(Phenyl)_3P]_2NiCl_2$, $[(Phenyl)_3P]_2PdCl_2$, $[(Phenyl)_2PCH_2]_2NiCl_2$, $[(Phenyl)_2PCH_2]_2CoBr_2$, $[(Phenyl)_2PCH_2CH_2CH_2P(Phenyl)_2]NiCl_2$, $[(Phenyl)_2PCH_2CH_2]_2NiCl_2$ und $[(Cyclohexyl)_3P]_2NiCl_2$.

Die Diarylzink-Verbindungen der Formel III sind entweder bekannt oder lassen sich analog bekannten Methoden herstellen (N.I. Sheverdina et al., Doklady Akad. SSSR 155, 623 (1964); engl.: 299).

Verbindungen der Formel II, in denen G für die Gruppe a steht, werden hier und im folgenden mit IIa bezeichnet und diejenigen mit der Gruppe b mit IIb.

Verbindungen der Formel IIa, in denen $R_2$ für Methyl, Ethyl, Isopropyl oder sek.Butyl steht und $R_1$ und E die für Formel II angegebenen Bedeutungen haben, und ein Verfahren zu ihrer Herstellung sind bekannt aus EP 180 539. Bei diesem Verfahren wird eine entsprechende 15-Hydroxy-$\Delta^{13,14}$-Verbindung mit einem Halogenierungsmittel in die 13$\beta$-Halogen-Verbindung überführt.

Die Verbindungen der Formel IIb

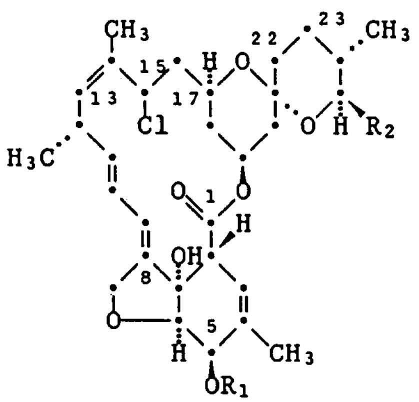

(IIb),

in denen

$R_1$ Wasserstoff oder eine OH-Schutzgruppe bedeutet; und

$R_2$ für Methyl, Ethyl, Isopropyl oder sek.Butyl steht,

sind neu und stellen ebenso wie das Verfahren zu ihrer Herstellung einen weiteren Aspekt der vorliegenden Erfindung dar. Die Herstellung von Verbindungen der Formel IIb erfolgt, indem man ein Milbemycin der Formel M mit einem die Bildung des entsprechenden 15-Chlor-$\Delta^{13,14}$-Derivats bewirkenden Chlorierungsmittel umsetzt.

Es ist aus EP 143 747 bereits bekannt, dass man bei Umsetzung von Milbemycinen der Formel M mit unterchloriger Säure (HOCl) oder Sulfurylchlorid ($SO_2Cl_2$) Verbindungen der Formel M'

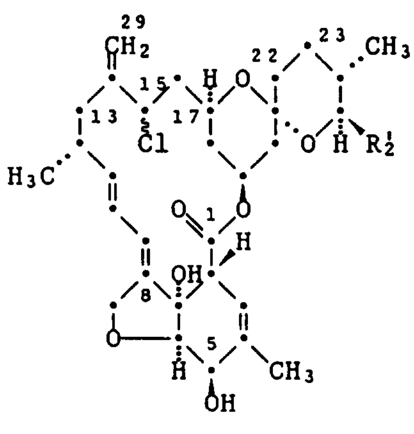

(M'),

worin $R_2'$ die für Formel M angegebenen Bedeutungen hat, erhält. Diese Reaktion stellt eine Chlorierung in 15-Position und die gleichzeitige Bildung einer exocyclischen Doppelbindung in 29-14-Stellung dar.

Ferner ist bekannt, dass die Chlorierung von Alkenyl- und Dialkenylverbindungen und von Cyclohexyliden mit tert.-Butylhypochlorit oder Chlor erfolgen kann (W. Sato et al., Chemistry Lett. 1982, 141-142; M. Yoshioka et al., Tetrahedron Lett. 21, 351-354).

Keines der bekannten Verfahren gibt jedoch einen Hinweis darauf, wie man zu den erfindungsgemässen Verbindungen der Formel IIb gelangen kann, welche endocylische Kohlenstoff-Kohlenstoff-Doppelbindungen aufweisen.

Es wurde nun gefunden, dass sich überraschenderweise Verbindungen der Formel IIb herstellen lassen, indem man eine Verbindung der Formel M mit tert.-Butylhypochlorit umsetzt. Dieses Verfahren bildet ebenfalls einen Aspekt der vorliegenden Erfindung.

Das Verfahren wird im allgemeinen in einem reaktions-inerten Lösungsmittel durchgeführt. Geeignete Lösungmittel sind beispielsweise Ether und etherartige Verbindungen, wie Dialkylether (Diethylether, Diisopropylether, tert.-Butylmethylether, Dimethoxyethan, Dioxan, Tetrahydrofuran oder Anisol); halogenierte Kohlenwasserstoffe, wie beispielsweise Chlorbenzol, Methylenchlorid oder Ethylenchlorid; oder Sulfoxide, wie Dimethylsulfoxid, wobei auch aromatische oder aliphatische Kohlenwasserstoffe, wie beispielsweise Benzol, Toluol, Xylole, Petrolether, Ligroin oder Cyclohexan anwesend sein können.

Die Reaktion wird im allgemeinen im Temperaturbereich -50 °C bis +50 °C, bevorzugt bei -10 °C bis +20 °C durchgeführt.

Ein weiteres Verfahren zur Herstellung von Verbindungen der Formel I besteht in der Einführung des Substituenten "Ph" in entsprechende 13-Acyloxy-$\Delta^{14,15}$-Derivate oder 15-Acyloxy-$\Delta^{13,14}$-Derivate durch Umsetzung dieser Derivate mit einer Triarylaluminium-Verbindung. Diese Umsetzung lässt sich analog der in EP 189 159 beschriebenen Methode, welche auch die Herstellung der acylierten Ausgangsverbindungen einschliesst, durchführen.

Ein weiterer Gegenstand der vorliegenden Erfindung ist somit ein Verfahren zur Herstellung der Verbindungen der Formel I, das darin besteht, dass man eine Verbindung der Formel V

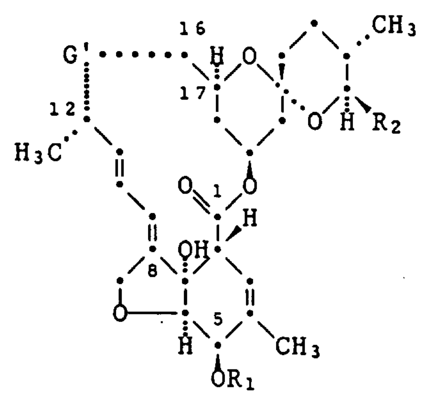

(V)

worin G' für eine der Gruppen a' oder b'

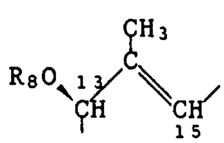 (a') oder 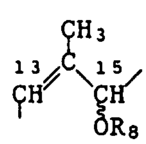 (b')

[= 13β-Ester-$\Delta^{14,15}$]     [= 15-Ester-$\Delta^{13,14}$]

steht, $R_8$ eine Acylgruppe repräsentiert, $R_1$ Wasserstoff oder vorzugsweise eine Silylgruppe bedeutet und $R_2$ die unter Formel I angegebene Bedeutung hat, mit einer Triarylaluminium-Verbindung der Formel VI

$Al(Ph)_3$     (VI)

behandelt, wobei Ph die unter Formel I angegebenen Bedeutungen hat, woraufhin man die $R_1$-Silylgruppe,

sofern freie 5-Hydroxy-Verbindungen erwünscht sind, hydrolytisch abspaltet.

Das Verfahren wird im allgemeinen in einem reaktions-inerten Lösungsmittel durchgeführt. Geeignete Lösungsmittel sind beispielsweise Ether und etherartige Verbindungen, wie Dialkylether (Diethylether, Diisopropylether, tert.-Butylmethylether, Dimethoxyethan, Dioxan, Tetrahydrofuran oder Anisol); halogenierte Kohlenwasserstoffe, wie beispielsweise Chlorbenzol, Methylenchlorid oder Ethylenchlorid; auch aromatische oder aliphatische Kohlenwasserstoffe, wie beispielsweise Benzol, Toluol, Xylole, Petrolether, Ligroin oder Cyclohexan können anwesend sein.

Es kann von Vorteil sein, wenn die Reaktion oder Teilschritte davon unter Schutzgasatmosphäre (beispielsweise Argon, Helium oder Stickstoff) und/oder in absoluten Lösungsmitteln durchgeführt werden. Gewünschtenfalls können Zwischenprodukte aus dem Reaktionsmedium isoliert und, falls erwünscht, vor der Weiterreaktion auf übliche Art und Weise gereinigt werden, z.B. durch Waschen, Digerieren, Extraktion, Umkristallisation, Chromatographie usw. Mann kann jedoch auch auf derartige Reinigungsschritte verzichten und diese erst mit entsprechenden Endprodukten durchführen.

Die Reaktion wird im allgemeinen im Temperaturbereich -100 °C bis 100 °C, bevorzugt bei -20 °C bis +60 °C durchgeführt. Die Triaryl-Aluminiumverbindung der Formel VI wird dabei in fester Form oder in einem reaktionsinerten Lösungsmittel wie z.B. Hexan, Toluol oder Benzol in mindestens equimolarer Menge zur Lösung der Verbindung der Formel V gegeben.

Nach erfolgter Reaktion kann die Silyl-Schutzgruppe durch Behandlung der Verbindungen der Formel I mit einer verdünnten Säure wie z.B. mit 1 proz. p-Toluolsulfonsäure in Methanol oder mit einer wässrigen HF-Lösung in Acetonitril im Temperaturbereich -20 °C bis 50 °C, bevorzugt bei 0 °C bis 30 °C, oder mit Pyridiniumfluorid in Pyridin wieder abgespalten werden.

Für $R_8$ kommen als Acyl-Gruppen beispielsweise in Frage: Formyl, Acetyl, Benzoyl, Ethoxycarbonyl oder $P(=O)(OAlkyl)_2$ wie $P(=O)(OEt)_2$, Alkylsulfonyl-Reste, vorzugsweise Niederalkylsulfonyl, insbesondere Mesyl, oder in begrenztem Umfang auch Tetrahydropyranyl.

Die unter den Umfang der Formel I fallenden 5-Keto-Milbemycine, in denen X für -C(O)- steht, lassen sich beispielsweise gewinnen, indem man Verbindungen der Formel I, in denen X für -CH(OH)- steht, mit einem zur Oxidation geeigneten Reagenz behandelt. Geeignete Oxydationsmittel sind beispielsweise aktiviertes Mangandioxyd, Oxalylchlorid/Dimethylsulfoxyd/Triethylamin oder Chromtrioxyd/Pyridin. Ein geeignetes Verfahren stellt auch die Oppenauer-Oxydation dar, bei welcher Verbindungen der Formel I, in denen X für -CH(OH)-steht, mit einem Keton, vorzugsweise Cyclohexanon oder Aceton, in Gegenwart eines Aluminiumalkoholats, vorzugsweise des Aluminiumisopropylats oder Aluminium-tert.-butylats, umgesetzt werden.

Die Oxydation wird zweckmässigerweise in einem inerten Lösungsmittel durchgeführt. Als geeignete Lösungsmittel kommen Alkane, wie beispielsweise Hexan, Heptan oder Oktan, aromatische Kohlenwasserstoffe, wie beispielsweise Benzol, Toluol oder Xylole, oder bevorzugt chlorierte Kohlenwasserstoffe, insbesondere Methylenchlorid, in Frage. Die Oxydation wird zweckmässigerweise bei Temperaturen von -80 °C bis +60 °C, vorzugsweise -60 °C bis +30 °C, durchgeführt.

Aus Verbindungen der Formeln I, in denen X für die Gruppe -C(O)-steht, lassen sich durch Reduktion auf an sich bekannte Weise wieder diejenigen Verbindungen gewinnen, in denen X für die Gruppe -CH(OH)- steht. Die Reduktion kann beispielsweise gemäss der Meerwein-Ponndorf-VerleyReduktion mit Aluminiumisopropylat in Isopropanol erfolgen.

Die Herstellung von Verbindungen der Formel I, in denen X für -C(=N-OR)-steht, kann beispielsweise erfolgen, indem man Verbindungen der Formeln I, in denen X für -C(O)- steht, mit Hydroxylamin oder einem seiner Salze umsetzt und gewünschtenfalls anschliessend den Substituenten R, wobei R die für Formel I angegebenen Bedeutungen mit Ausnahme von Wasserstoff hat, einführt, oder indem man die Umsetzung mit einer Verbindung der Formel $NH_2$-OR, worin R die für Formel I angegebenen Bedeutungen mit Ausnahme von Wasserstoff hat, oder einem Salz davon vornimmt. Als Salze kommen beispielsweise solche der vorgenannten Aminoverbindungen mit Schwefelsäure, Salpetersäure und vor allem Salzsäure in Betracht. Die Reaktion wird zweckmässigerweise in einem geeigneten Lösungsmittel ausgeführt, z.B. einem Niederalkanol, wie Methanol, Ethanol, Propanol; einer etherartigen Verbindung, wie Tetrahydrofuran oder Dioxan; einer aliphatischen Carbonsäure, wie Essigsäure oder Propionsäure; Wasser oder in Gemischen dieser Lösungsmittel untereinander oder mit anderen üblichen reaktionsinerten Lösungsmitteln. Die Reaktionstemperaturen können in weiten Bereichen variieren. Man arbeitet zweckmässigerweise etwa im Bereich von +10° bis +100 °C. Wird Hydroxylamin in Form eines seiner Salze, z.B. als Hydrochlorid eingesetzt, so ist es vorteilhaft, wenn man zum Abfangen der Säure eine der für solche Zwecke üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, n-$C_3H_7ONa$ usw. Bevorzugt wird Triethylamin.

Die Verbindungen der Formel I eignen sich ausgezeichnet zur Bekämpfung von Schädlingen an Tieren und Pflanzen in allen ihren Entwicklungsstadien, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen der Formel I sind auch einsetzbar gegen Hygiene-Schädlinge, insbesondere solche der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen der Formel I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten); der Ordnungen Hemiptera; Heteroptera und Thysanoptera; sowie bei den pflanzenfressenden Insekten der Ordnungen Lepidoptera, Coleoptera, Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Gattungen Meloidogyne, Heterodera, Pratylenchus, Ditylenchus, Radopholus, Rizoglyphus und andere.

Ferner sind die Verbindungen gegen Helminthen in allen Entwicklungsstadien wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Wenn die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie

auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich. Ueber geschlossenen Kultur-Anbauflächen werden sie zweckmässigerweise in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsauren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"1986 International Mc Cutcheon's Emulsifiers and Detergents" The Manufacturing Confectioner Publishing Co., Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Beispiel A 1: Herstellung von 5-O-tert.Butyldimethylsilyl-15-chlor-$\Delta^{13,14}$-Milbemycin $A_4$

Zu einer Lösung von 1 g (1,52 mmol) 5-0-tert.Butyldimethylsilyl-Milbemycin $A_4$ in 2 ml Dichlormethan und 8 ml Diethylether werden bei -10°C unter Rühren 200 $\mu$l (182 mg; 1,52 mmol) tert.Butylhypochlorit zugegeben. Nach 1 Stunde Rühren bei Raumtemperatur wird das Lösungsmittel abgedampft. Die Chromatographie des Rohprodukts (80 g Kieselgel; Laufmittel: Essigester/Hexan 1:16) ergibt (neben 321 mg 5-O-tert.Butyldimethylsilyl-15-chlor-$\Delta^{14,29}$-Milbemycin $A_4$) 486 mg der Titelverbindung 5-O-tert.Butyldimethylsilyl-15-chlor-$\Delta^{13,14}$-Milbemycin $A_4$.

$^1$H-NMR (300 MHz, $CDCl_3$, TMS)
3,05 ppm (m) ($C_{12}H$)
4,42 ppm (m) ($C_{15}H$)
4,87 ppm (m) ($C_{19}H$)
5,23 ppm (d, J = 10 Hz) ($C_{13}H$)
Massenspektrum FD m/e: 690 ($M^+$, $C_{38}H_{59}ClO_7Si$)

### Beispiel A2: Herstellung von 15-Chlor-$\Delta^{13,14}$-Milbemycin $A_4$

Zu einer Lösung von 203 mg (0,375 mmol) Milbemycin $A_4$ in 5 ml Diethylether werden bei O°C unter Rühren 0,049 ml (45 mg; 0,412 mmol) tert.-Butylhypochlorit zugegeben. Nach 2 Stunden Rühren bei Raumtemperatur wird das Lösungsmittel abgedampft. Die Chromatographie des Rohprodukts (20 g Kieselgel; Laufmittel: Aceton/Dichlormethan 1:5O) ergibt (neben 55 mg 15-Chlor-$\Delta^{14,29}$-Milbemycin $A_4$) 97 mg der Titelverbindung 15-Chlor-$\Delta^{13,14}$-Milbemycin $A_4$.

$^1$H-NMR (3OO MHz, $CDCl_3$, TMS)
3,06 ppm (m) ($C_{12}H$)
4,39 ppm (dd, J = 4 und 12 Hz) ($C_{15}H$)
4,87 ppm (m) ($C_{19}H$)
5,23 ppm (m) ($C_{13}H$)

Massenspektrum FD m/e: 576 ($M^+$, $C_{32}H_{45}ClO_7$)

Beispiel H1: Herstellung von 13β-Phenyl-milbemycin $A_4$

a) Herstellung von Diphenylzink:
Zu 818 mg (6 mmol) Zinkchlorid werden bei 0°C unter Rühren in Argonatmosphäre 6 ml (12 mmol) einer 2 M Lösung von Phenylmagnesiumchlorid in Tetrahydrofuran zugegeben. Nach 16 Stunden Rühren bei Raumtemperatur werden 5 ml Toluol zugegeben. Die Lösung wird 10 Minuten gerührt und anschliessend ohne Rühren aufbewahrt.
b) Herstellung von 5-O-tert.Butyldimethylsilyl-13β-phenyl-Milbemycin $A_4$:
Zu einer Lösung von 150 mg (0,204 mmol) 5-O-tert.Butyldimethylsilyl-13β-brom-Milbemycin $A_4$ und 13,4 mg (0,020 mmol) Bis-(triphenylphosphin)-nickel(II)chlorid [(Dichlor-bis(triphenylphosphin)-Nickel; $NiCl_2$ -(Phenyl$_3$P)$_2$] in 1 ml Toluol werden unter Rühren in Argonatmosphäre bei Raumtemperatur 4 ml der nach a) erhaltenen Lösung zugegeben. Nach 10 Minuten bei Raumtemperatur wird mit Aether, 2M Natrium-Kalium-Tartrat-Lösung und Kieselguhr aufgearbeitet. Die Chromatographie des Rohprodukts (Kieselgel 20 g; Laufmittel: Essigester/Hexan 1:12) und HPLC (Laufmittel: Diethylether/Hexan 1:6) ergibt 38 mg 5-O-tert.Butyldimethylsilyl-13β-phenyl-milbemycin $A_4$. $^1$H-NMR (300 MHz, $CDCl_3$, TMS)
2,94 ppm (m) ($C_{12}H$)
3,07 ppm (d, J = 10 Hz) ($C_{13}H$)
7,15-7,30 ppm (m) (Phenyl).
Massenspektrum (FD) m/e: 732 ($M^+$, $C_{44}H_{64}O_7Si$).
c) Herstellung von 13β-Phenyl-milbemycin $A_4$:
34 mg (O,O46 mmol) 5-O-tert.Butyldimethylsilyl-13β-phenyl-milbemycin $A_4$ werden mit 2 ml einer 40 %igen wässrigen Lösung von HF in Acetonitril (5 : 95) während 2 Stunden bei Raumtemperatur behandelt. Die Aufarbeitung in Diethylether mit 5 %iger wässriger $NaHCO_3$-Lösung und Chromatographie an Kieselgel (Essigester/Hexan 1:2) liefert 27 mg 13β-Phenylmilbemycin $A_4$.
$^1$H-NMR (300 MHz, $CDCl_3$, TMS)
2,84 ppm (m) ($C_{12}H$)
3,10 ppm (d, J = 10 Hz) ($C_{13}H$)
7,16-7,35 ppm (m) (Phenyl)
Massenspektrum (FD) m/e: 618 ($M^+$, $C_{38}H_{50}O_7$).

Beispiel H2: Herstellung von 13β-(4-Biphenylyl)-milbemycin $A_4$

Analog Beispiel H1 aber mit 5-O-tert.Butyldimethylsilyl-15-chlor-$\Delta^{13,14}$-milbemycin $A_4$ statt 5-O-tert.Butyldimethylsilyl-13β-brom-milbemycin $A_4$ wird 13β-(4-Biphenylyl)-milbemycin $A_4$ hergestellt.
$^1$H-NMR (300 MHz, $CDCl_3$, TMS)
2,93 ppm (m) ($C_{12}H$)
3,17 ppm (d, J = 10 Hz) ($C_{13}H$)
7,25-7,61 ppm (m) (Biphenyl)
Massenspektrum (FD) m/e: 694 ($C_{44}H_{54}O_7$).

Beispiel H3: Herstellung von 13β-(3-Methoxyphenyl)-milbemycin $A_4$

Analog Beispiel H2 wird 13β-(3-Methoxyphenyl)-milbemycin $A_4$ hergestellt.
$^1$H-NMR (300 MHz, $CDCl_3$, TMS)
2,83 ppm (m) ($C_{12}H$)
3,10 ppm (d, J = 10 Hz) ($C_{13}H$)
3,83 ppm (s) ($CH_3O$)
6,74-6,89 ppm (m) (aromatisch)
Massenspektrum (FD) m/e: 648 ($C_{39}H_{52}O_8$).

Analog den vorstehend beschriebenen Methoden werden auch folgende Verbindungen hergestellt:

Beispiel H4: 13β-(3,4-Dimethoxyphenyl)-milbemycin $A_4$

$^1$H-NMR (300 MHz, $CDCl_3$, TMS)
2,80 ppm (m) ($C_{12}H$)
3,06 ppm (d, J = 10 Hz) ($C_{13}H$)

3,86 ppm (s) ($CH_3O$)
3,88 ppm (s) ($CH_3O$)
6,70-6,83 ppm (m) (aromatisch)
Massenspektrum (FD) m/e: 678 ($C_{40}H_{54}O_9$).

Beispiel H5: 13$\beta$-(4-Phenoxyphenyl)-milbemycin $A_4$

$^1$H-NMR (3OO MHz, $CDCl_3$, TMS)
2,80 ppm (m) ($C_{12}H$)
3,07 ppm (d, J = 10 Hz) ($C_{13}H$)
6,88-7,34 ppm (m) (aromatisch)
Massenspektrum (FD) m/e: 710 ($C_{44}H_{54}O_8$).

Beispiel H6: 13$\beta$-(2-Methylphenyl)-milbemycin $A_4$

$^1$H-NMR (300 MHz, $CDCl_3$, TMS)
2,91 ppm (m, $C_{12}H$)
3,36 ppm (d, J = 10 Hz) ($C_{13}H$)
7,08 - 7,30 (m) (aromatisch)
Massenspektrum (FD) m/e: 632 ($C_{39}H_{52}O_7$).

Beispiel H7: 13$\beta$-(4-Methylphenyl)-milbemycin $A_4$

$^1$H-NMR (300 MHz, $CDCl_3$, TMS)
2,84 ppm (m, $C_{12}H$)
3,08 ppm (d, J = 10 Hz) ($C_{13}H$)
7,27 ppm (m) (aromatisch)
Massenspektrum (FD) m/e: 632 ($C_{39}H_{52}O_7$).

Beispiel H8: 13$\beta$-(3-Methylphenyl)-milbemycin $A_4$

$^1$H-NMR (300 MHz, $CDCl_3$, TMS)
2,33 ppm (s) ($CH_3$-aromatisch)
2,86 ppm (m) ($C_{12}H$)
3,08 ppm (d, J = 10 Hz) ($C_{13}H$)
6,99 - 7,27 ppm (m) (aromatisch)
Massenspektrum (FD) m/e: 632 ($C_{39}H_{52}O_7$).

Beispiel H9: 13$\beta$-(4-Methoxyphenyl)-milbemycin $A_4$

$^1$H-NMR (300 MHz, $CDCl_3$, TMS)
2,81 ppm (m) ($C_{12}H$)
3,07 ppm (d, J = 10 Hz) ($C_{13}H$)
3,79 ppm (s) ($CH_3O$)
6,84 ppm (d, J = 8 Hz) und 7,12 ppm (d, J = 8 Hz) (aromatisch).
Massenspektrum (FD) m/e: 648 ($C_{39}H_{52}O_8$).

Beispiel H10: 13$\beta$-(2-Methoxyphenyl)-milbemycin $A_4$

$^1$H-NMR (300 MHz, $CDCl_3$, TMS)
2,83 ppm (m) ($C_{12}H$)
3,81 ppm (s) ($CH_3O$)
6,83-7,28 ppm (m) (aromatisch).

Beispiel H11: 13$\beta$-(3-Biphenylyl)-milbemycin $A_4$

$^1$H-NMR (300 MHz, $CDCl_3$, TMS)
2,94 ppm (m) ($C_{12}H$)

3,19 ppm (d, J = 10 Hz) ($C_{13}H$)
3,98 ppm (d, J = 6 Hz) ($C_6H$)
7,17-7,60 ppm (m) (aromatisch).

Beispiel H12: 13β-Phenyl-milbemycin $A_3$

$^1$H-NMR (300 MHz, $CDCl_3$, TMS)
2,77 ppm (m, $C_{12}H$)
3,01 ppm (d, J = 10 Hz) ($C_{13}H$)
3,18 ppm (m) ($C_{25}H$)
7,09-7,23 ppm (m) (aromatisch).

Analog zu den beschriebenen Arbeitsweisen werden auch die nachfolgend zusammen mit Verbindungen der vorgängigen Beispiele genannten Verbindungen der Formel I hergestellt:

Tabelle 1

Typische Vertreter von Verbindungen der Formel I, worin X für $-CH(OR_1)-$ steht und $R_1$ Wasserstoff ist.

| Verb. Nr. | $R_2$ | Ph |
|---|---|---|
| 1.1 | $CH_3$ | 2-Methylphenyl |
| 1.2 | $C_2H_5$ | 2-Methylphenyl |
| 1.3 | $C_3H_7$-iso | 2-Methylphenyl |
| 1.4 | $C_4H_9$-sek | 2-Methylphenyl |
| 1.5 | $CH_3$ | 4-Methoxyphenyl |
| 1.6 | $C_2H_5$ | 4-Methoxyphenyl |
| 1.7 | $C_3H_7$-iso | 4-Methoxyphenyl |
| 1.8 | $C_4H_9$-sek | 4-Methoxyphenyl |
| 1.9 | $CH_3$ | 2,3-Dimethylphenyl |
| 1.10 | $C_2H_5$ | 2,3-Dimethylphenyl |
| 1.11 | $C_3H_7$-iso | 2,3-Dimethylphenyl |
| 1.12 | $C_4H_9$-sek | 2,3-Dimethylphenyl |
| 1.13 | $CH_3$ | 3-(2-Methoxyäthoxy)-phenyl |
| 1.14 | $C_2H_5$ | 3-(2-Methoxyäthoxy)-phenyl |
| 1.15 | $C_3H_7$-iso | 3-(2-Methoxyäthoxy)-phenyl |
| 1.16 | $C_4H_9$-sek | 3-(2-Methoxyäthoxy)-phenyl |
| 1.17 | $CH_3$ | Phenyl |
| 1.18 | $C_2H_5$ | Phenyl |
| 1.19 | $C_3H_7$-iso | Phenyl |
| 1.20 | $C_4H_9$-sek | Phenyl |
| 1.21 | $CH_3$ | 2-n-Butylphenyl |
| 1.22 | $C_2H_5$ | 2-n-Butylphenyl |
| 1.23 | $C_3H_7$-iso | 2-n-Butylphenyl |
| 1.24 | $C_4H_9$-sek | 2-n-Butylphenyl |
| 1.25 | $CH_3$ | 3,4-Dimethoxyphenyl |
| 1.26 | $C_2H_5$ | 3,4-Dimethoxyphenyl |
| 1.27 | $C_3H_7$-iso | 3,4-Dimethoxyphenyl |
| 1.28 | $C_4H_9$-sek | 3,4-Dimethoxyphenyl |
| 1.29 | $CH_3$ | 4-Isopropylphenyl |
| 1.30 | $C_2H_5$ | 4-Isopropylphenyl |
| 1.31 | $C_3H_7$-iso | 4-Isopropylphenyl |
| 1.32 | $C_4H_9$-sek | 4-Isopropylphenyl |
| 1.33 | $CH_3$ | 4-(Aethoxymethyl)-phenyl |
| 1.34 | $C_2H_5$ | 4-(Aethoxymethyl)-phenyl |
| 1.35 | $C_3H_7$-iso | 4-(Aethoxymethyl)-phenyl |
| 1.36 | $C_4H_9$-sek | 4-(Aethoxymethyl)-phenyl |
| 1.37 | $CH_3$ | 4-Methylphenyl |
| 1.38 | $C_2H_5$ | 4-Methylphenyl |

Tabelle 1 (Fortsetzung)

| Verb. Nr. | $R_2$ | Ph |
|---|---|---|
| 1.39 | $C_3H_7$-iso | 4-Methylphenyl |
| 1.40 | $C_4H_9$-sek | 4-Methylphenyl |
| 1.41 | $CH_3$ | 2-n-Propoxyphenyl |
| 1.42 | $C_2H_5$ | 2-n-Propoxyphenyl |
| 1.43 | $C_3H_7$-iso | 2-n-Propoxyphenyl |
| 1.44 | $C_4H_9$-sek | 2-n-Propoxyphenyl |
| 1.45 | $CH_3$ | 2,3,5,6-Tetramethoxyphenyl |
| 1.46 | $C_2H_5$ | 2,3,5,6-Tetramethoxyphenyl |
| 1.47 | $C_3H_7$-iso | 2,3,5,6-Tetramethoxyphenyl |
| 1.48 | $C_4H_9$-sek | 2,3,5,6-Tetramethoxyphenyl |
| 1.49 | $CH_3$ | 3-Vinylphenyl |
| 1.50 | $C_2H_5$ | 3-Vinylphenyl |
| 1.51 | $C_3H_7$-iso | 3-Vinylphenyl |
| 1.52 | $C_4H_9$-sek | 3-Vinylphenyl |
| 1.53 | $CH_3$ | 4-n-Propylphenyl |
| 1.54 | $C_2H_5$ | 4-n-Propylphenyl |
| 1.55 | $C_3H_7$-iso | 4-n-Propylphenyl |
| 1.56 | $C_4H_9$-sek | 4-n-Propylphenyl |
| 1.57 | $CH_3$ | 3-Aethoxyphenyl |
| 1.58 | $C_2H_5$ | 3-Aethoxyphenyl |
| 1.59 | $C_3H_7$-iso | 3-Aethoxyphenyl |
| 1.60 | $C_4H_9$-sek | 3-Aethoxyphenyl |
| 1.61 | $CH_3$ | 2-Aethylphenyl |
| 1.62 | $C_2H_5$ | 2-Aethylphenyl |
| 1.63 | $C_3H_7$-iso | 2-Aethylphenyl |
| 1.64 | $C_4H_9$-sek | 2-Aethylphenyl |
| 1.65 | $CH_3$ | 2-Methyl-4-methoxyphenyl |
| 1.66 | $C_2H_5$ | 2-Methyl-4-methoxyphenyl |
| 1.67 | $C_3H_7$-iso | 2-Methyl-4-methoxyphenyl |
| 1.68 | $C_4H_9$-sek | 2-Methyl-4-methoxyphenyl |
| 1.69 | $CH_3$ | 2-(Methoxymethoxy)-phenyl |
| 1.70 | $C_2H_5$ | 2-(Methoxymethoxy)-phenyl |
| 1.71 | $C_3H_7$-iso | 2-(Methoxymethoxy)-phenyl |
| 1.72 | $C_4H_9$-sek | 2-(Methoxymethoxy)-phenyl |

Tabelle 1 (Fortsetzung)

| Verb. Nr. | $R_2$ | Ph |
|---|---|---|
| 1.73 | $CH_3$ | 4-Aethylphenyl |
| 1.74 | $C_2H_5$ | 4-Aethylphenyl |
| 1.75 | $C_3H_7$-iso | 4-Aethylphenyl |
| 1.76 | $C_4H_9$-sek | 4-Aethylphenyl |
| 1.77 | $CH_3$ | 2,4-Dimethoxyphenyl |
| 1.78 | $C_2H_5$ | 2,4-Dimethoxyphenyl |
| 1.79 | $C_3H_7$-iso | 2,4-Dimethoxyphenyl |
| 1.80 | $C_4H_9$-sek | 2,4-Dimethoxyphenyl |
| 1.81 | $CH_3$ | 3-(2-Methoxyäthyl)-phenyl |
| 1.82 | $C_2H_5$ | 3-(2-Methoxyäthyl)-phenyl |
| 1.83 | $C_3H_7$-iso | 3-(2-Methoxyäthyl)-phenyl |
| 1.84 | $C_4H_9$-sek | 3-(2-Methoxyäthyl)-phenyl |
| 1.85 | $CH_3$ | 4-tert.-Butylphenyl |
| 1.86 | $C_2H_5$ | 4-tert.-Butylphenyl |
| 1.87 | $C_3H_7$-iso | 4-tert.-Butylphenyl |
| 1.88 | $C_4H_9$-sek | 4-tert.-Butylphenyl |
| 1.89 | $CH_3$ | 2-Methoxyphenyl |
| 1.90 | $C_2H_5$ | 2-Methoxyphenyl |
| 1.91 | $C_3H_7$-iso | 2-Methoxyphenyl |
| 1.92 | $C_4H_9$-sek | 2-Methoxyphenyl |
| 1.93 | $CH_3$ | 2,4,6-Trimethylphenyl |
| 1.94 | $C_2H_5$ | 2,4,6-Trimethylphenyl |
| 1.95 | $C_3H_7$-iso | 2,4,6-Trimethylphenyl |
| 1.96 | $C_4H_9$-sek | 2,4,6-Trimethylphenyl |
| 1.97 | $CH_3$ | 4-(2-n-Propoxyäthoxy)-phenyl |
| 1.98 | $C_2H_5$ | 4-(2-n-Propoxyäthoxy)-phenyl |
| 1.99 | $C_3H_7$-iso | 4-(2-n-Propoxyäthoxy)-phenyl |
| 1.100 | $C_4H_9$-sek | 4-(2-n-Propoxyäthoxy)-phenyl |
| 1.101 | $CH_3$ | 3-n-Propylphenyl |
| 1.102 | $C_2H_5$ | 3-n-Propylphenyl |
| 1.103 | $C_3H_7$-iso | 3-n-Propylphenyl |
| 1.104 | $C_4H_9$-sek | 3-n-Propylphenyl |
| 1.105 | $CH_3$ | 4-Allylphenyl |
| 1.106 | $C_2H_5$ | 4-Allylphenyl |
| 1.107 | $C_3H_7$-iso | 4-Allylphenyl |
| 1.108 | $C_4H_9$-sek | 4-Allylphenyl |
| 1.109 | $CH_3$ | 3,4-Dimethylphenyl |
| 1.110 | $C_2H_5$ | 3,4-Dimethylphenyl |
| 1.111 | $C_3H_7$-iso | 3,4-Dimethylphenyl |
| 1.112 | $C_4H_9$-sek | 3,4-Dimethylphenyl |

Tabelle 1 (Fortsetzung)

| Verb. Nr. | $R_2$ | Ph |
|---|---|---|
| 1.113 | $CH_3$ | 2-Aethoxyphenyl |
| 1.114 | $C_2H_5$ | 2-Aethoxyphenyl |
| 1.115 | $C_3H_7$-iso | 2-Aethoxyphenyl |
| 1.116 | $C_4H_9$-sek | 2-Aethoxyphenyl |
| 1.117 | $CH_3$ | 3-n-Propoxyphenyl |
| 1.118 | $C_2H_5$ | 3-n-Propoxyphenyl |
| 1.119 | $C_3H_7$-iso | 3-n-Propoxyphenyl |
| 1.120 | $C_4H_9$-sek | 3-n-Propoxyphenyl |
| 1.121 | $CH_3$ | 2-Isopropylphenyl |
| 1.122 | $C_2H_5$ | 2-Isopropylphenyl |
| 1.123 | $C_3H_7$-iso | 2-Isopropylphenyl |
| 1.124 | $C_4H_9$-sek | 2-Isopropylphenyl |
| 1.125 | $CH_3$ | 2,3-Dimethoxyphenyl |
| 1.126 | $C_2H_5$ | 2,3-Dimethoxyphenyl |
| 1.127 | $C_3H_7$-iso | 2,3-Dimethoxyphenyl |
| 1.128 | $C_4H_9$-sek | 2,3-Dimethoxyphenyl |
| 1.129 | $CH_3$ | 3-Methylphenyl |
| 1.130 | $C_2H_5$ | 3-Methylphenyl |
| 1.131 | $C_3H_7$-iso | 3-Methylphenyl |
| 1.132 | $C_4H_9$-sek | 3-Methylphenyl |
| 1.133 | $CH_3$ | 2-(Aethoxymethoxy)-phenyl |
| 1.134 | $C_2H_5$ | 2-(Aethoxymethoxy)-phenyl |
| 1.135 | $C_3H_7$-iso | 2-(Aethoxymethoxy)-phenyl |
| 1.136 | $C_4H_9$-sek | 2-(Aethoxymethoxy)-phenyl |
| 1.137 | $CH_3$ | 3-Isobutylphenyl |
| 1.138 | $C_2H_5$ | 3-Isobutylphenyl |
| 1.139 | $C_3H_7$-iso | 3-Isobutylphenyl |
| 1.140 | $C_4H_9$-sek | 3-Isobutylphenyl |
| 1.141 | $CH_3$ | 4-n-Propoxyphenyl |
| 1.142 | $C_2H_5$ | 4-n-Propoxyphenyl |
| 1.143 | $C_3H_7$-iso | 4-n-Propoxyphenyl |
| 1.144 | $C_4H_9$-sek | 4-n-Propoxyphenyl |
| 1.145 | $CH_3$ | 2,4-Dimethylphenyl |
| 1.146 | $C_2H_5$ | 2,4-Dimethylphenyl |
| 1.147 | $C_3H_7$-iso | 2,4-Dimethylphenyl |
| 1.148 | $C_4H_9$-sek | 2,4-Dimethylphenyl |
| 1.149 | $CH_3$ | 3-Methoxyphenyl |
| 1.150 | $C_2H_5$ | 3-Methoxyphenyl |
| 1.151 | $C_3H_7$-iso | 3-Methoxyphenyl |
| 1.152 | $C_4H_9$-sek | 3-Methoxyphenyl |

Tabelle 1 (Fortsetzung)

| Verb. Nr. | $R_2$ | Ph |
|---|---|---|
| 1.153 | $C_3H_7$-iso | 4-(tert.-Butoxymethyl)-phenyl |
| 1.154 | $C_4H_9$-sek | 4-(tert.-Butoxymethyl)-phenyl |
| 1.155 | $CH_3$ | 4-(tert.-Butoxymethyl)-phenyl |
| 1.156 | $C_2H_5$ | 4-(tert.-Butoxymethyl)-phenyl |
| 1.157 | $C_3H_7$-iso | 3-Isopropylphenyl |
| 1.158 | $C_4H_9$-sek | 3-Isopropylphenyl |
| 1.159 | $CH_3$ | 3-Isopropylphenyl |
| 1.160 | $C_2H_5$ | 3-Isopropylphenyl |
| 1.161 | $C_3H_7$-iso | 3,5-Dimethoxyphenyl |
| 1.162 | $C_4H_9$-sek | 3,5-Dimethoxyphenyl |
| 1.163 | $CH_3$ | 3,5-Dimethoxyphenyl |
| 1.164 | $C_2H_5$ | 3,5-Dimethoxyphenyl |
| 1.165 | $C_3H_7$-iso | 2-n-Propylphenyl |
| 1.166 | $C_4H_9$-sek | 2-n-Propylphenyl |
| 1.167 | $CH_3$ | 2-n-Propylphenyl |
| 1.168 | $C_2H_5$ | 2-n-Propylphenyl |
| 1.169 | $C_3H_7$-iso | 2-(Methoxymethyl)-phenyl |
| 1.170 | $C_4H_9$-sek | 2-(Methoxymethyl)-phenyl |
| 1.171 | $CH_3$ | 2-(Methoxymethyl)-phenyl |
| 1.172 | $C_2H_5$ | 2-(Methoxymethyl)-phenyl |
| 1.173 | $C_3H_7$-iso | 4-Aethoxyphenyl |
| 1.174 | $C_4H_9$-sek | 4-Aethoxyphenyl |
| 1.175 | $CH_3$ | 4-Aethoxyphenyl |
| 1.176 | $C_2H_5$ | 4-Aethoxyphenyl |
| 1.177 | $C_3H_7$-iso | 2,4,6-Trimethoxyphenyl |
| 1.178 | $C_4H_9$-sek | 2,4,6-Trimethoxyphenyl |
| 1.179 | $CH_3$ | 2,4,6-Trimethoxyphenyl |
| 1.180 | $C_2H_5$ | 2,4,6-Trimethoxyphenyl |
| 1.181 | $C_3H_7$-iso | 3-Aethylphenyl |
| 1.182 | $C_4H_9$-sek | 3-Aethylphenyl |
| 1.183 | $CH_3$ | 3-Aethylphenyl |
| 1.184 | $C_2H_5$ | 3-Aethylphenyl |
| 1.185 | $CH_3$ | 3,5-Dimethylphenyl |
| 1.186 | $C_2H_5$ | 3,5-Dimethylphenyl |
| 1.187 | $C_3H_7$-iso | 3,5-Dimethylphenyl |
| 1.188 | $C_4H_9$-sek | 3,5-Dimethylphenyl |
| 1.189 | $CH_3$ | 3-Methoxy-4-methylphenyl |
| 1.190 | $C_2H_5$ | 3-Methoxy-4-methylphenyl |
| 1.191 | $C_3H_7$-iso | 3-Methoxy-4-methylphenyl |
| 1.192 | $C_4H_9$-sek | 3-Methoxy-4-methylphenyl |

Tabelle 1 (Fortsetzung)

| Verb. Nr. | $R_2$ | Ph |
|---|---|---|
| 1.193 | $CH_3$ | 4-(3-Methyl-4-methoxyphenoxy)-phenyl |
| 1.194 | $C_2H_5$ | 4-(3-Methyl-4-methoxyphenoxy)-phenyl |
| 1.195 | $C_3H_7$-iso | 4-(3-Methyl-4-methoxyphenoxy)-phenyl |
| 1.196 | $C_4H_9$-sek | 4-(3-Methyl-4-methoxyphenoxy)-phenyl |
| 1.197 | $CH_3$ | 3-Biphenylyl |
| 1.198 | $C_2H_5$ | 3-Biphenylyl |
| 1.199 | $C_3H_7$-iso | 3-Biphenylyl |
| 1.200 | $C_4H_9$-sek | 3-Biphenylyl |
| 1.201 | $CH_3$ | 2'-Aethoxy-3-biphenylyl |
| 1.202 | $C_2H_5$ | 2'-Aethoxy-3-biphenylyl |
| 1.203 | $C_3H_7$-iso | 2'-Aethoxy-3-biphenylyl |
| 1.204 | $C_4H_9$-sek | 2'-Aethoxy-3-biphenylyl |
| 1.205 | $CH_3$ | 3',4',5'-Trimethoxy-4-biphenylyl |
| 1.206 | $C_2H_5$ | 3',4',5'-Trimethoxy-4-biphenylyl |
| 1.207 | $C_3H_7$-iso | 3',4',5'-Trimethoxy-4-biphenylyl |
| 1.208 | $C_4H_9$-sek | 3',4',5'-Trimethoxy-4-biphenylyl |
| 1.209 | $CH_3$ | 3-Phenoxyphenyl |
| 1.210 | $C_2H_5$ | 3-Phenoxyphenyl |
| 1.211 | $C_3H_7$-iso | 3-Phenoxyphenyl |
| 1.212 | $C_4H_9$-sek | 3-Phenoxyphenyl |
| 1.213 | $CH_3$ | 4'-Methoxy-3-biphenylyl |
| 1.214 | $C_2H_5$ | 4'-Methoxy-3-biphenylyl |
| 1.215 | $C_3H_7$-iso | 4'-Methoxy-3-biphenylyl |
| 1.216 | $C_4H_9$-sek | 4'-Methoxy-3-biphenylyl |
| 1.217 | $CH_3$ | 4-(3-Methoxyphenoxy)-phenyl |
| 1.218 | $C_2H_5$ | 4-(3-Methoxyphenoxy)-phenyl |
| 1.219 | $C_3H_7$-iso | 4-(3-Methoxyphenoxy)-phenyl |
| 1.220 | $C_4H_9$-sek | 4-(3-Methoxyphenoxy)-phenyl |
| 1.221 | $CH_3$ | 2'-Methyl-4-biphenylyl |
| 1.222 | $C_2H_5$ | 2'-Methyl-4-biphenylyl |
| 1.223 | $C_3H_7$-iso | 2'-Methyl-4-biphenylyl |
| 1.224 | $C_4H_9$-sek | 2'-Methyl-4-biphenylyl |
| 1.225 | $CH_3$ | 3'-n-Propoxy-3-biphenylyl |
| 1.226 | $C_2H_5$ | 3'-n-Propoxy-3-biphenylyl |
| 1.227 | $C_3H_7$-iso | 3'-n-Propoxy-3-biphenylyl |
| 1.228 | $C_4H_9$-sek | 3'-n-Propoxy-3-biphenylyl |
| 1.229 | $CH_3$ | 4-(4-Methylphenoxy)-phenyl |
| 1.230 | $C_2H_5$ | 4-(4-Methylphenoxy)-phenyl |
| 1.231 | $C_3H_7$-iso | 4-(4-Methylphenoxy)-phenyl |
| 1.232 | $C_4H_9$-sek | 4-(4-Methylphenoxy)-phenyl |

Tabelle 1 (Fortsetzung)

| Verb. Nr. | $R_2$ | Ph |
|---|---|---|
| 1.233 | $CH_3$ | 3'-Methyl-3-biphenylyl |
| 1.234 | $C_2H_5$ | 3'-Methyl-3-biphenylyl |
| 1.235 | $C_3H_7$-iso | 3'-Methyl-3-biphenylyl |
| 1.236 | $C_4H_9$-sek | 3'-Methyl-3-biphenylyl |
| 1.237 | $CH_3$ | 4-(2-Methylphenoxy)-phenyl |
| 1.238 | $C_2H_5$ | 4-(2-Methylphenoxy)-phenyl |
| 1.239 | $C_3H_7$-iso | 4-(2-Methylphenoxy)-phenyl |
| 1.240 | $C_4H_9$-sek | 4-(2-Methylphenoxy)-phenyl |
| 1.241 | $CH_3$ | 3',4'-Dimethyl-4-biphenylyl |
| 1.242 | $C_2H_5$ | 3',4'-Dimethyl-4-biphenylyl |
| 1.243 | $C_3H_7$-iso | 3',4'-Dimethyl-4-biphenylyl |
| 1.244 | $C_4H_9$-sek | 3',4'-Dimethyl-4-biphenylyl |
| 1.245 | $CH_3$ | 3-(2-Methoxyphenoxy)-phenyl |
| 1.246 | $C_2H_5$ | 3-(2-Methoxyphenoxy)-phenyl |
| 1.247 | $C_3H_7$-iso | 3-(2-Methoxyphenoxy)-phenyl |
| 1.248 | $C_4H_9$-sek | 3-(2-Methoxyphenoxy)-phenyl |
| 1.249 | $CH_3$ | 2'-Aethyl-3-biphenylyl |
| 1.250 | $C_2H_5$ | 2'-Aethyl-3-biphenylyl |
| 1.251 | $C_3H_7$-iso | 2'-Aethyl-3-biphenylyl |
| 1.252 | $C_4H_9$-sek | 2'-Aethyl-3-biphenylyl |
| 1.253 | $CH_3$ | 3'-Methoxy-5'-methyl-4-biphenylyl |
| 1.254 | $C_2H_5$ | 3'-Methoxy-5'-methyl-4-biphenylyl |
| 1.255 | $C_3H_7$-iso | 3'-Methoxy-5'-methyl-4-biphenylyl |
| 1.256 | $C_4H_9$-sek | 3'-Methoxy-5'-methyl-4-biphenylyl |
| 1.257 | $CH_3$ | 4-Phenoxyphenyl |
| 1.258 | $C_2H_5$ | 4-Phenoxyphenyl |
| 1.259 | $C_3H_7$-iso | 4-Phenoxyphenyl |
| 1.260 | $C_4H_9$-sek | 4-Phenoxyphenyl |
| 1.261 | $CH_3$ | 3',5'-Dimethoxy-3-biphenylyl |
| 1.262 | $C_2H_5$ | 3',5'-Dimethoxy-3-biphenylyl |
| 1.263 | $C_3H_7$-iso | 3',5'-Dimethoxy-3-biphenylyl |
| 1.264 | $C_4H_9$-sek | 3',5'-Dimethoxy-3-biphenylyl |
| 1.265 | $CH_3$ | 3-(3-Methylphenoxy)-phenyl |
| 1.266 | $C_2H_5$ | 3-(3-Methylphenoxy)-phenyl |
| 1.267 | $C_3H_7$-iso | 3-(3-Methylphenoxy)-phenyl |
| 1.268 | $C_4H_9$-sek | 3-(3-Methylphenoxy)-phenyl |
| 1.269 | $CH_3$ | 4-(3,4-Dimethoxyphenoxy)-phenyl |
| 1.270 | $C_2H_5$ | 4-(3,4-Dimethoxyphenoxy)-phenyl |

Tabelle 1 (Fortsetzung)

| Verb. Nr. | $R_2$ | Ph |
|---|---|---|
| 1.271 | $C_3H_7$-iso | 4-(3,4-Dimethoxyphenoxy)-phenyl |
| 1.272 | $C_4H_9$-sek | 4-(3,4-Dimethoxyphenoxy)-phenyl |
| 1.273 | $CH_3$ | 4-Biphenylyl |
| 1.274 | $C_2H_5$ | 4-Biphenylyl |
| 1.275 | $C_3H_7$-iso | 4-Biphenylyl |
| 1.276 | $C_4H_9$-sek | 4-Biphenylyl |
| 1.277 | $CH_3$ | 2'-Methyl-3-biphenylyl |
| 1.278 | $C_2H_5$ | 2'-Methyl-3-biphenylyl |
| 1.279 | $C_3H_7$-iso | 2'-Methyl-3-biphenylyl |
| 1.280 | $C_4H_9$-sek | 2'-Methyl-3-biphenylyl |
| 1.281 | $CH_3$ | 3'-Aethoxy-4-biphenylyl |
| 1.282 | $C_2H_5$ | 3'-Aethoxy-4-biphenylyl |
| 1.283 | $C_3H_7$-iso | 3'-Aethoxy-4-biphenylyl |
| 1.284 | $C_4H_9$-sek | 3'-Aethoxy-4-biphenylyl |
| 1.285 | $CH_3$ | 4-(4-Methoxyphenoxy)-phenyl |
| 1.286 | $C_2H_5$ | 4-(4-Methoxyphenoxy)-phenyl |
| 1.287 | $C_3H_7$-iso | 4-(4-Methoxyphenoxy)-phenyl |
| 1.288 | $C_4H_9$-sek | 4-(4-Methoxyphenoxy)-phenyl |
| 1.289 | $CH_3$ | 3-(2-Methylphenoxy)-phenyl |
| 1.290 | $C_2H_5$ | 3-(2-Methylphenoxy)-phenyl |
| 1.291 | $C_3H_7$-iso | 3-(2-Methylphenoxy)-phenyl |
| 1.292 | $C_4H_9$-sek | 3-(2-Methylphenoxy)-phenyl |
| 1.293 | $CH_3$ | 4-(3,4-Dimethylphenoxy)-phenyl |
| 1.294 | $C_2H_5$ | 4-(3,4-Dimethylphenoxy)-phenyl |
| 1.295 | $C_3H_7$-iso | 4-(3,4-Dimethylphenoxy)-phenyl |
| 1.296 | $C_4H_9$-sek | 4-(3,4-Dimethylphenoxy)-phenyl |
| 1.297 | $CH_3$ | 2'-Methoxy-3-biphenylyl |
| 1.298 | $C_2H_5$ | 2'-Methoxy-3-biphenylyl |
| 1.299 | $C_3H_7$-iso | 2'-Methoxy-3-biphenylyl |
| 1.300 | $C_4H_9$-sek | 2'-Methoxy-3-biphenylyl |
| 1.301 | $CH_3$ | 4'-Methyl-3-biphenylyl |
| 1.302 | $C_2H_5$ | 4'-Methyl-3-biphenylyl |
| 1.303 | $C_3H_7$-iso | 4'-Methyl-3-biphenylyl |
| 1.304 | $C_4H_9$-sek | 4'-Methyl-3-biphenylyl |
| 1.305 | $CH_3$ | 4'-n-Propyl-4-biphenylyl |
| 1.306 | $C_2H_5$ | 4'-n-Propyl-4-biphenylyl |
| 1.307 | $C_3H_7$-iso | 4'-n-Propyl-4-biphenylyl |
| 1.308 | $C_4H_9$-sek | 4'-n-Propyl-4-biphenylyl |
| 1.309 | $CH_3$ | 4-(3-Methylphenoxy)-phenyl |
| 1.310 | $C_2H_5$ | 4-(3-Methylphenoxy)-phenyl |
| 1.311 | $C_3H_7$-iso | 4-(3-Methylphenoxy)-phenyl |
| 1.312 | $C_4H_9$-sek | 4-(3-Methylphenoxy)-phenyl |

Tabelle 1 (Fortsetzung)

| Verb. Nr. | $R_2$ | Ph |
|---|---|---|
| 1.313 | $CH_3$ | 2'-Methoxy-4-biphenylyl |
| 1.314 | $C_2H_5$ | 2'-Methoxy-4-biphenylyl |
| 1.315 | $C_3H_7$-iso | 2'-Methoxy-4-biphenylyl |
| 1.316 | $C_4H_9$-sek | 2'-Methoxy-4-biphenylyl |
| 1.317 | $CH_3$ | 4'-n-Propoxy-4-biphenylyl |
| 1.318 | $C_2H_5$ | 4'-n-Propoxy-4-biphenylyl |
| 1.319 | $C_3H_7$-iso | 4'-n-Propoxy-4-biphenylyl |
| 1.320 | $C_4H_9$-sek | 4'-n-Propoxy-4-biphenylyl |
| 1.321 | $CH_3$ | 3',4'-Dimethyl-3-biphenylyl |
| 1.322 | $C_2H_5$ | 3',4'-Dimethyl-3-biphenylyl |
| 1.323 | $C_3H_7$-iso | 3',4'-Dimethyl-3-biphenylyl |
| 1.324 | $C_4H_9$-sek | 3',4'-Dimethyl-3-biphenylyl |
| 1.325 | $CH_3$ | 3'-Methyl-4'-methoxy-4-biphenylyl |
| 1.326 | $C_2H_5$ | 3'-Methyl-4'-methoxy-4-biphenylyl |
| 1.327 | $C_3H_7$-iso | 3'-Methyl-4'-methoxy-4-biphenylyl |
| 1.328 | $C_4H_9$-sek | 3'-Methyl-4'-methoxy-4-biphenylyl |
| 1.329 | $CH_3$ | 3-(4-Methylphenoxy)-phenyl |
| 1.330 | $C_2H_5$ | 3-(4-Methylphenoxy)-phenyl |
| 1.331 | $C_3H_7$-iso | 3-(4-Methylphenoxy)-phenyl |
| 1.332 | $C_4H_9$-sek | 3-(4-Methylphenoxy)-phenyl |
| 1.333 | $CH_3$ | 3',4',5'-Trimethyl-3-biphenylyl |
| 1.334 | $C_2H_5$ | 3',4',5'-Trimethyl-3-biphenylyl |
| 1.335 | $C_3H_7$-iso | 3',4',5'-Trimethyl-3-biphenylyl |
| 1.336 | $C_4H_9$-sek | 3',4',5'-Trimethyl-3-biphenylyl |
| 1.337 | $CH_3$ | 2'-Aethoxy-4-biphenylyl |
| 1.338 | $C_2H_5$ | 2'-Aethoxy-4-biphenylyl |
| 1.339 | $C_3H_7$-iso | 2'-Aethoxy-4-biphenylyl |
| 1.340 | $C_4H_9$-sek | 2'-Aethoxy-4-biphenylyl |
| 1.341 | $CH_3$ | 4'-Methyl-4-biphenylyl |
| 1.342 | $C_2H_5$ | 4'-Methyl-4-biphenylyl |
| 1.343 | $C_3H_7$-iso | 4'-Methyl-4-biphenylyl |
| 1.344 | $C_4H_9$-sek | 4'-Methyl-4-biphenylyl |
| 1.345 | $CH_3$ | 3',4'-Dimethoxy-3-biphenylyl |
| 1.346 | $C_2H_5$ | 3',4'-Dimethoxy-3-biphenylyl |
| 1.347 | $C_3H_7$-iso | 3',4'-Dimethoxy-3-biphenylyl |
| 1.348 | $C_4H_9$-sek | 3',4'-Dimethoxy-3-biphenylyl |
| 1.349 | $CH_3$ | 3-(3,5-Dimethoxyphenoxy)-phenyl |
| 1.350 | $C_2H_5$ | 3-(3,5-Dimethoxyphenoxy)-phenyl |
| 1.351 | $C_3H_7$-iso | 3-(3,5-Dimethoxyphenoxy)-phenyl |
| 1.352 | $C_4H_9$-sek | 3-(3,5-Dimethoxyphenoxy)-phenyl |
| 1.353 | $CH_3$ | 3',4'-Dimethoxy-4-biphenylyl |
| 1.354 | $C_2H_5$ | 3',4'-Dimethoxy-4-biphenylyl |
| 1.355 | $C_3H_7$-iso | 3',4'-Dimethoxy-4-biphenylyl |
| 1.356 | $C_4H_9$-sek | 3',4'-Dimethoxy-4-biphenylyl |

Tabelle 1 (Fortsetzung)

| Verb. Nr. | $R_2$ | Ph |
|---|---|---|
| 1.357 | $CH_3$ | 4'-Aethoxy-3-biphenylyl |
| 1.358 | $C_2H_5$ | 4'-Aethoxy-3-biphenylyl |
| 1.359 | $C_3H_7$-iso | 4'-Aethoxy-3-biphenylyl |
| 1.360 | $C_4H_9$-sek | 4'-Aethoxy-3-biphenylyl |
| 1.361 | $CH_3$ | 3'-Methyl-4-biphenylyl |
| 1.362 | $C_2H_5$ | 3'-Methyl-4-biphenylyl |
| 1.363 | $C_3H_7$-iso | 3'-Methyl-4-biphenylyl |
| 1.364 | $C_4H_9$-sek | 3'-Methyl-4-biphenylyl |
| 1.365 | $CH_3$ | 3'-Methoxy-3-biphenylyl |
| 1.366 | $C_2H_5$ | 3'-Methoxy-3-biphenylyl |
| 1.367 | $C_3H_7$-iso | 3'-Methoxy-3-biphenylyl |
| 1.368 | $C_4H_9$-sek | 3'-Methoxy-3-biphenylyl |
| 1.369 | $CH_3$ | 3',5'-Dimethyl-4-biphenyl |
| 1.370 | $C_2H_5$ | 3',5'-Dimethyl-4-biphenyl |
| 1.371 | $C_3H_7$-iso | 3',5'-Dimethyl-4-biphenyl |
| 1.372 | $C_4H_9$-sek | 3',5'-Dimethyl-4-biphenyl |
| 1.373 | $CH_3$ | 4'-Methoxy-4-biphenylyl |
| 1.374 | $C_2H_5$ | 4'-Methoxy-4-biphenylyl |
| 1.375 | $C_3H_7$-iso | 4'-Methoxy-4-biphenylyl |
| 1.376 | $C_4H_9$-sek | 4'-Methoxy-4-biphenylyl |
| 1.377 | $CH_3$ | 3'-Methoxy-4-biphenylyl |
| 1.378 | $C_2H_5$ | 3'-Methoxy-4-biphenylyl |
| 1.379 | $C_3H_7$-iso | 3'-Methoxy-4-biphenylyl |
| 1.380 | $C_4H_9$-sek | 3'-Methoxy-4-biphenylyl |
| 1.381 | $CH_3$ | 5-Methyl-3-biphenylyl |
| 1.382 | $C_2H_5$ | 5-Methyl-3-biphenylyl |
| 1.383 | $C_3H_7$-iso | 5-Methyl-3-biphenylyl |
| 1.384 | $C_4H_9$-sek | 5-Methyl-3-biphenylyl |
| 1.385 | $CH_3$ | 2-Methoxy-4-(3-Methylphenoxy)-phenyl |
| 1.386 | $C_2H_5$ | 2-Methoxy-4-(3-Methylphenoxy)-phenyl |
| 1.387 | $C_3H_7$-iso | 2-Methoxy-4-(3-Methylphenoxy)-phenyl |
| 1.388 | $C_4H_9$-sek | 2-Methoxy-4-(3-Methylphenoxy)-phenyl |
| 1.389 | $CH_3$ | 4'-Methoxy-2-methyl-4-biphenylyl |
| 1.390 | $C_2H_5$ | 4'-Methoxy-2-methyl-4-biphenylyl |
| 1.391 | $C_3H_7$-iso | 4'-Methoxy-2-methyl-4-biphenylyl |
| 1.392 | $C_4H_9$-sek | 4'-Methoxy-2-methyl-4-biphenylyl |
| 1.393 | $CH_3$ | 5-Methoxy-3-biphenylyl |
| 1.394 | $C_2H_5$ | 5-Methoxy-3-biphenylyl |
| 1.395 | $C_3H_7$-iso | 5-Methoxy-3-biphenylyl |
| 1.396 | $C_4H_9$-sek | 5-Methoxy-3-biphenylyl |

Tabelle 2

Typische Vertreter von Verbindungen der Formel I, worin X für -C(O)- steht.

| Verb. Nr. | $R_2$ | Ph |
|---|---|---|
| 2.1 | $CH_3$ | 4-Biphenylyl |
| 2.2 | $C_2H_5$ | 4-Biphenylyl |
| 2.3 | $C_3H_7$-iso | 4-Biphenylyl |
| 2.4 | $C_4H_9$-sek | 4-Biphenylyl |
| 2.5 | $CH_3$ | 3-Methylphenyl |
| 2.6 | $C_2H_5$ | 3-Methylphenyl |
| 2.7 | $C_3H_7$-iso | 3-Methylphenyl |
| 2.8 | $C_4H_9$-sek | 3-Methylphenyl |
| 2.9 | $CH_3$ | 3-Biphenylyl |
| 2.10 | $C_2H_5$ | 3-Biphenylyl |
| 2.11 | $C_3H_7$-iso | 3-Biphenylyl |
| 2.12 | $C_4H_9$-sek | 3-Biphenylyl |
| 2.13 | $CH_3$ | Phenyl |
| 2.14 | $C_2H_5$ | Phenyl |
| 2.15 | $C_3H_7$-iso | Phenyl |
| 2.16 | $C_4H_9$-sek | Phenyl |
| 2.17 | $CH_3$ | 2-Methylphenyl |
| 2.18 | $C_2H_5$ | 2-Methylphenyl |
| 2.19 | $C_3H_7$-iso | 2-Methylphenyl |
| 2.20 | $C_4H_9$-sek | 2-Methylphenyl |
| 2.21 | $CH_3$ | 4-Methoxyphenyl |
| 2.22 | $C_2H_5$ | 4-Methoxyphenyl |
| 2.23 | $C_3H_7$-iso | 4-Methoxyphenyl |
| 2.24 | $C_4H_9$-sek | 4-Methoxyphenyl |
| 2.25 | $CH_3$ | 3-Methoxyphenyl |
| 2.26 | $C_2H_5$ | 3-Methoxyphenyl |
| 2.27 | $C_3H_7$-iso | 3-Methoxyphenyl |
| 2.28 | $C_4H_9$-sek | 3-Methoxyphenyl |
| 2.29 | $CH_3$ | 2-Methoxyphenyl |
| 2.30 | $C_2H_5$ | 2-Methoxyphenyl |
| 2.31 | $C_3H_7$-iso | 2-Methoxyphenyl |
| 2.32 | $C_4H_9$-sek | 2-Methoxyphenyl |
| 2.33 | $CH_3$ | 3,4-Dimethoxyphenyl |
| 2.34 | $C_2H_5$ | 3,4-Dimethoxyphenyl |
| 2.35 | $C_3H_7$-iso | 3,4-Dimethoxyphenyl |
| 2.36 | $C_4H_9$-sek | 3,4-Dimethoxyphenyl |
| 2.37 | $CH_3$ | 4-Phenoxyphenyl |
| 2.38 | $C_2H_5$ | 4-Phenoxyphenyl |
| 2.39 | $C_3H_7$-iso | 4-Phenoxyphenyl |
| 2.40 | $C_4H_9$-sek | 4-Phenoxyphenyl |
| 2.41 | $CH_3$ | 4-Methylphenyl |
| 2.42 | $C_2H_5$ | 4-Methylphenyl |
| 2.43 | $C_3H_7$-iso | 4-Methylphenyl |
| 2.44 | $C_4H_9$-sek | 4-Methylphenyl |

Tabelle 3

Typische Vertreter von Verbindungen der Formel I, worin X für -C(=N-OR)- steht und R Wasserstoff bedeutet.

| Verb. Nr. | $R_2$ | Ph |
|---|---|---|
| 3.1 | $CH_3$ | 4-Biphenylyl |
| 3.2 | $C_2H_5$ | 4-Biphenylyl |
| 3.3 | $C_3H_7$-iso | 4-Biphenylyl |
| 3.4 | $C_4H_9$-sek | 4-Biphenylyl |
| 3.5 | $CH_3$ | 3-Methylphenyl |
| 3.6 | $C_2H_5$ | 3-Methylphenyl |
| 3.7 | $C_3H_7$-iso | 3-Methylphenyl |
| 3.8 | $C_4H_9$-sek | 3-Methylphenyl |
| 3.9 | $CH_3$ | 3-Biphenylyl |
| 3.10 | $C_2H_5$ | 3-Biphenylyl |
| 3.11 | $C_3H_7$-iso | 3-Biphenylyl |
| 3.12 | $C_4H_9$-sek | 3-Biphenylyl |
| 3.13 | $CH_3$ | Phenyl |
| 3.14 | $C_2H_5$ | Phenyl |
| 3.15 | $C_3H_7$-iso | Phenyl |
| 3.16 | $C_4H_9$-sek | Phenyl |
| 3.17 | $CH_3$ | 2-Methylphenyl |
| 3.18 | $C_2H_5$ | 2-Methylphenyl |
| 3.19 | $C_3H_7$-iso | 2-Methylphenyl |
| 3.20 | $C_4H_9$-sek | 2-Methylphenyl |
| 3.21 | $CH_3$ | 4-Methoxyphenyl |
| 3.22 | $C_2H_5$ | 4-Methoxyphenyl |
| 3.23 | $C_3H_7$-iso | 4-Methoxyphenyl |
| 3.24 | $C_4H_9$-sek | 4-Methoxyphenyl |
| 3.25 | $CH_3$ | 3-Methoxyphenyl |
| 3.26 | $C_2H_5$ | 3-Methoxyphenyl |
| 3.27 | $C_3H_7$-iso | 3-Methoxyphenyl |
| 3.28 | $C_4H_9$-sek | 3-Methoxyphenyl |
| 3.29 | $CH_3$ | 2-Methoxyphenyl |
| 3.30 | $C_2H_5$ | 2-Methoxyphenyl |
| 3.31 | $C_3H_7$-iso | 2-Methoxyphenyl |
| 3.32 | $C_4H_9$-sek | 2-Methoxyphenyl |
| 3.33 | $CH_3$ | 3,4-Dimethoxyphenyl |
| 3.34 | $C_2H_5$ | 3,4-Dimethoxyphenyl |
| 3.35 | $C_3H_7$-iso | 3,4-Dimethoxyphenyl |
| 3.36 | $C_4H_9$-sek | 3,4-Dimethoxyphenyl |
| 3.37 | $CH_3$ | 4-Phenoxyphenyl |
| 3.38 | $C_2H_5$ | 4-Phenoxyphenyl |
| 3.39 | $C_3H_7$-iso | 4-Phenoxyphenyl |
| 3.40 | $C_4H_9$-sek | 4-Phenoxyphenyl |
| 3.41 | $CH_3$ | 4-Methylphenyl |
| 3.42 | $C_2H_5$ | 4-Methylphenyl |
| 3.43 | $C_3H_7$-iso | 4-Methylphenyl |
| 3.44 | $C_4H_9$-sek | 4-Methylphenyl |

Formulierungsbeispiele für den Wirkstoff der Formel I
(% = Gewichtsprozent)

| Spritzpulver | a) | b) | c) |
|---|---|---|---|
| Wirkstoff aus Beispielen H1-H12 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus Beispielen H1-H12 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus Beispielen H1-H12 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Extruder Granulat | |
|---|---|
| Wirkstoff aus Beispielen H1-H12 | 10 % |
| Na-Ligninsulfonat | 2 % |
| Carboximethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Tabletten bzw. Boli | | |
|---|---|---|
| I | Ein Wirkstoff aus Beispielen H1-H12 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist.<br>Maisstärke<br>mikrokrist. Cellulose<br>Magnesiumstearat | 22,50 %<br>17,00 %<br>16,50 %<br>1,00 % |
| Alle 4 Hilfsstoffe gut mischen | | |

Phasen I und II mischen und zu Tabletten oder Boli verpressen.

Injektabiles

A. Oeliges Vehikel (langsame Freisetzung)

| | |
|---|---|
| Ein Wirkstoff aus Beispielen H1-H12 | 0,1-1,0 g |
| Erdnussöl | ad 100 ml |
| Ein Wirkstoff aus Beispielen H1-H12 | 0,1-1,0 g |
| Sesamöl | ad 100 ml |

Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 µm sterilfiltriert.

B. Wassermischbares Lösungsmittel (mittlere Freisetzungsgeschwindigkeit)

| | |
|---|---|
| Ein Wirkstoff aus Beispielen H1-H12 | 0,1-1,0 g |
| 4-Hydroxymethyl-1,3-dioxolan (Glycerol Formal) | 40 g |
| 1,2-Propandiol | ad 100 ml |
| Ein Wirkstoff aus Beispielen H1-H12 | 0,1-1,0 g |
| Glycerindimethylketal | 40 g |
| 1,2-Propandiol | ad 100 ml |

Herstellung: Der Wirkstoff wird in einem Teil des Lösungsmittels unter Rühren gelöst, auf das Sollvolumen aufgefüllt und durch ein geeignetes Membranfilter mit 0.22 µm sterilfiltriert.

C. Wässriges Solubilisat (rasche Freisetzung)

| | |
|---|---|
| Ein Wirkstoff aus Beispielen H1-H12 | 0,1-1,0 g |
| Polyäthoxyliertes Ricinusöl (40 Aethylenoxideinheiten)* | 10 g |
| 1,2-Propandiol | 20 g |
| Benzylalkohol | 1 g |
| Aqua ad injekt. | ad 100 ml |

* Im Handel erhältlich unter der Bezeichnung CREMOPHOR® EL (BASF AG);

| | |
|---|---|
| Ein Wirkstoff aus Beispielen H1-H12 | 0,1-1,0 g |
| Polyäthoxyliertes Sorbitanmonooleat (20 Aethylenoxideinheiten)** | 8 g |
| 4-Hydroxymethyl-1,3-dioxolan (Glycerol Formal) | 20 g |
| Benzylalkohol | 1 g |
| Aqua ad injekt. | ad 100 ml |

** Im Handel erhältlich unter der Bezeichnung TWEEN® 80 (ICI);

Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 µm Porendurchmesser.

Die wässrigen Systeme können bevorzugterweise auch für die orale und/oder intraruminale Applikation eingesetzt werden.

Biologische Beispiele

## B-1. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50 °C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I, beispielsweise von den Beispielen H1 bis H12, bewirken eine vollständige Abtötung bei 250 ppm.

## B-2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von 1,0 µg pro Zecke gelöst ist. Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I, beispielsweise von den Beispielen H1 bis H12, zeigen eine $IR_{90}$ bei einer Aufwandmenge von 5 µg/g.

## B-3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit Verbindungen der Formeln I, beispielsweise denen der Beispiele H1 bis H12 bei 1 mg/kg behandelt werden, zeigen im Vergleich zu unbehandelten, aber infizierten Vergleichsgruppen keinen Nematodenbefall (= komplette Reduktion der Wurmeier im Kot).

B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formeln I, wie beispielsweise jene der Beispiele H1 bis H12, bewirken in diesem Test bei niedriger Konzentration bereits nach einem Tag eine vollständige Abtötung sämtlicher Larven.

B-5. Milbizide Wirkung gegen Dermanyssus gallinae

2 bis 3 ml einer Testlösung mit 100 ppm Aktivsubstanz werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen H1 bis H12 bewirken bei der angegebenen Konzentration eine Abtötung der Milben.

**Patentansprüche**

**Patentansprüche für folgende Vertragsstaaten : AT, BE, CH, DE, FR, GB, IT, LI, LU, NL, SE**

**1.** Verbindungen der Formel I

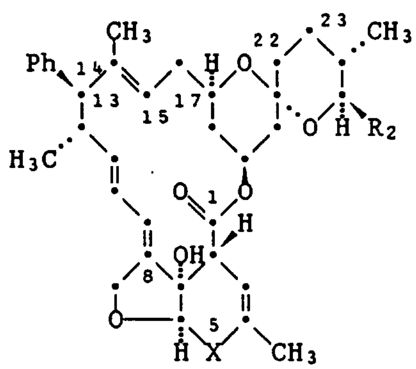

(I)

in welcher

X eine der Gruppen $-CH(OR_1)-$, $-C(O)-$ oder $-C(=N-OR)-$ repräsentiert;

$R_1$ Wasserstoff oder eine OH-Schutzgruppe bedeutet;

R für Wasserstoff, eine OH-Schutzgruppe, eine Alkyl- oder Cycloalkylgruppe steht;

$R_2$ für Methyl, Ethyl, Isopropyl oder sek.Butyl steht; und

Ph einen durch $R_a$, $R_b$, $R_c$ und $R_d$ substituierten Phenylring darstellt,

wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**2.** Verbindungen der Formel I nach Anspruch 1, worin X $-CH(OR_1)-$ und $R_1$ Wasserstoff oder eine Schutzgruppe bedeutet, $R_2$ für Methyl, Ethyl, Isopropyl oder sek.-Butyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**3.** Verbindungen der Formel I nach Anspruch 1, worin X -CH($OR_1$)- und $R_1$ Wasserstoff bedeutet, $R_2$ für Methyl, Ethyl, Isopropyl oder sek.-Butyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**4.** Verbindungen der Formel I nach Anspruch 1, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**5.** Verbindungen der Formel I nach Anspruch 1, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy oder $C_2$-$C_{10}$-Alkoxyalkoxy stehen.

**6.** Verbindungen der Formel I nach Anspruch 1, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**7.** Verbindungen der Formel I nach Anspruch 1, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_3$-Alkyl, oder $C_1$-$C_3$-Alkoxy stehen, mit der Massgabe, dass höchstens zwei der Substituenten $R_a$, $R_b$, $R_c$ und $R_d$ eine andere Bedeutung als Wasserstoff haben.

**8.** Verbindungen der Formel I nach Anspruch 1, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, Phenyl oder Phenoxy stehen, mit der Massgabe, dass höchstens zwei der Substituenten $R_a$, $R_b$, $R_c$ und $R_d$ eine andere Bedeutung als Wasserstoff haben.

**9.** Verbindungen der Formel I nach Anspruch 1, worin X -CH($OR_1$)- und $R_1$ Wasserstoff bedeutet, $R_2$ für Methyl oder Ethyl, steht; und Ph für unsubstituiertes oder durch Methyl, Methoxy, Phenyl oder Phenoxy monosubstituiertes oder durch Methyl oder Methoxy disubstituiertes oder durch Methyl trisubstituiertes Phenyl steht.

**10.** Verbindungen der Formel I nach Anspruch 1, worin X -CH($OR_1$)- und $R_1$ Wasserstoff bedeutet, $R_2$ für Ethyl steht; und Ph für unsubstituiertes oder durch Methyl, Methoxy, Phenyl oder Phenoxy monosubstituiertes oder durch Methyl oder Methoxy disubstituiertes oder durch Methyl trisubstituiertes Phenyl steht.

**11.** Verbindungen der Formel I nach Anspruch 1, worin X -CH($OR_1$)- und $R_1$ Wasserstoff bedeutet, $R_2$ für Methyl oder insbesondere für Ethyl steht; und Ph für unsubstituiertes oder durch Methyl, Methoxy, Phenyl oder Phenoxy monosubstituiertes oder durch Methoxy disubstituiertes Phenyl steht.

**12.** Eine Verbindung der Formel I ausgewählt aus der Gruppe:
13$\beta$-Phenyl-milbemycin $A_4$,
13$\beta$-(2-Methoxyphenyl)-milbemycin $A_4$,
13$\beta$-(3-Methoxyphenyl)-milbemycin $A_4$,
13$\beta$-(4-Methoxyphenyl)-milbemycin $A_4$,
13$\beta$-(2-Methylphenyl)-milbemycin $A_4$,
13$\beta$-(4-Biphenylyl)-milbemycin $A_4$,

13β-(4-Phenoxyphenyl)-milbemycin $A_4$ und
13β-(3,4-Dimethoxyphenyl)-milbemycin $A_4$.

**13.** Eine Verbindung der Formel I ausgewählt aus der Gruppe:
13β-Phenyl-milbemycin $A_3$,
13β-(2-Methylphenyl)-milbemycin $A_4$ und
13β-(4-Methylphenyl)-milbemycin $A_4$.

**14.** Verfahren zur Herstellung von Verbindungen der Formel I, dadurch gekennzeichnet, dass man eine Verbindung der Formel II

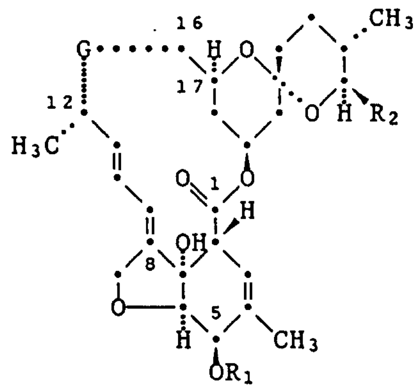

(II)

worin G für eine der Gruppen a oder b

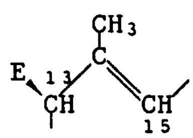

(a) oder

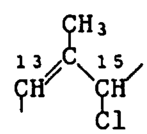

(b)

[= 13β-Halogen-$\Delta^{14,15}$] [= 15-Chlor-$\Delta^{13,14}$]

steht, $R_1$ Wasserstoff oder eine OH-Schutzgruppe bedeutet, $R_2$ die für Formel I angegebenen Bedeutungen hat und E für Chlor, Brom oder Jod steht, mit einer Diarylzink-Verbindung der Formel III

Ph-Zn-Ph (III),

worin Ph die für Formel I angegebene Bedeutung hat, in Gegenwart eines Uebergangsmetallsalzes umsetzt und gewünschtenfalls, wenn $R_1$ für eine OH-Schutzgruppe steht, diese Schutzgruppe hydrolytisch abspaltet.

**15.** Verfahren nach Anspruch 14, dadurch gekennzeichnet, dass man die Umsetzung in Gegenwart eines Uebergangsmetallsalzes, welches als metallische Komponente Fe, Ru, Os, Co, Rh, Ir, Ni, Pd oder Pt enthält, durchführt.

**16.** Verfahren nach Anspruch 15, dadurch gekennzeichnet, dass man die Umsetzung in Gegenwart eines Uebergangsmetallsalzes, welches als metallische Komponente Co, Ni oder Pd enthält, durchführt.

**17.** Verfahren nach Anspruch 14, dadurch gekennzeichnet, dass man als Uebergangsmetallsalz $NiCl_2$, $CoCl_2$, $[(Phenyl)_3P]_2NiCl_2$, $[(Phenyl)_3P]_2PdCl_2$, $[(Phenyl)_2PCH_2]_2NiCl_2$, $[(Phenyl)_2PCH_2]_2CoBr_2$, $[(Phenyl)_2PCH_2CH_2CH_2P(Phenyl)_2]NiCl_2$, $[(Phenyl)_2PCH_2CH_2]_2NiCl_2$ oder $[(Cyclohexyl)_3P]_2NiCl_2$ verwendet.

**18.** Verfahren zur Herstellung von Verbindungen der Formel IIb

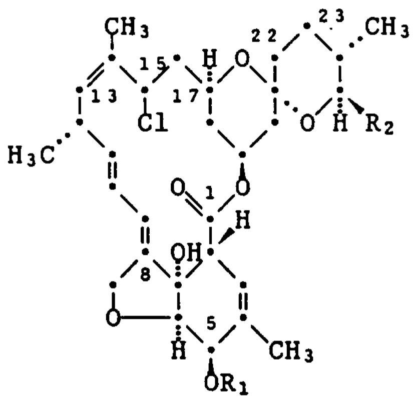

(IIb)

in denen

$R_1$ Wasserstoff oder eine OH-Schutzgruppe bedeutet; und

$R_2$ für Methyl, Ethyl, Isopropyl oder sek.Butyl steht,

dadurch gekennzeichnet, dass man ein Milbemycin der Formel M

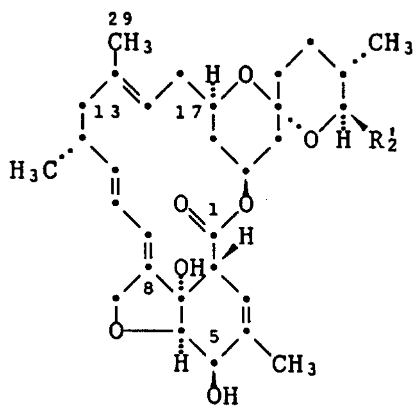

(M)

worin $R_2'$ für Methyl, Ethyl, Isopropyl oder sek.-Butyl steht, mit einem die Bildung des entsprechenden 15-Chlor-$\Delta^{13,14}$-Derivats bewirkenden Chlorierungsmittel umsetzt.

**19.** Verfahren nach Anspruch 18, dadurch gekennzeichnet, dass man als Chlorierungsmittel tert.-Butylhypochlorit verwendet.

**20.** Verbindungen der Formel IIb

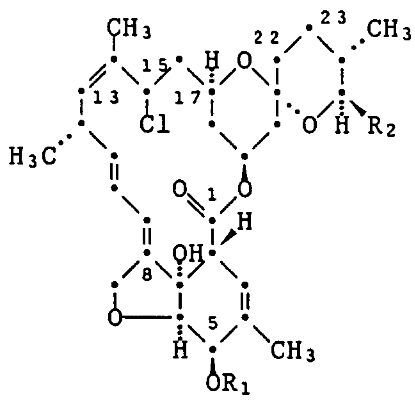

(IIb),

in denen

$R_1$ Wasserstoff oder eine OH-Schutzgruppe bedeutet; und

$R_2$ für Methyl, Ethyl, Isopropyl oder sek.Butyl steht.

**21.** Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I gemäss Anspruch 1 enthält.

**22.** Verfahren zur Bekämpfung von Schädlingen, dadurch gekennzeichnet, dass man eine pestizid-wirksame Menge mindestens einer Verbindung der Formel I gemäss Anspruch 1 auf oder in die Wirtspflanzen oder sonstigen Lebensräume der Schädlinge mit Ausnahme menschlicher oder tierischer Körper appliziert.

**23.** Verfahren nach Anspruch 22, dadurch gekennzeichnet, dass die zu bekämpfenden Schädlinge Tiere befallende Endo- oder Ektoparasiten sind.

**24.** Verfahren nach Anspruch 22, dadurch gekennzeichnet, dass die zu bekämpfenden Schädlinge pflanzenschädigende Parasiten sind.

**25.** Verfahren nach Anspruch 22, dadurch gekennzeichnet, dass es sich bei den Schädlingen um parasitäre Nematoden handelt.

**26.** Verbindungen der Formel I gemäss Anspruch 1 zur Verwendung als antiparasitäres Mittel gegen Tiere befallende Endo- und Ektoparasiten.

**27.** Verwendung einer Verbindung der Formel I zur Herstellung eines Arzneimittels zur Bekämpfung von Tiere befallenden Endo- und Ektoparasiten.

**Patentansprüche für folgende Vertragsstaaten : ES, GR**

1. Verfahren zur Herstellung von Verbindungen der Formel I

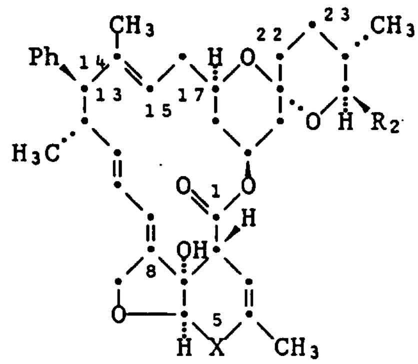

(I)

in welcher

X eine der Gruppen $-CH(OR_1)-$, -C(O)- oder -C(=N-OR)- repräsentiert;

$R_1$ Wasserstoff oder eine OH-Schutzgruppe bedeutet;

R für Wasserstoff, eine OH-Schutzgruppe, eine Alkyl- oder Cycloalkylgruppe steht;

$R_2$ für Methyl, Ethyl, Isopropyl oder sek.Butyl steht; und

Ph einen durch $R_a$, $R_b$, $R_c$ und $R_d$ substituierten Phenylring darstellt,

wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen, dadurch gekennzeichnet, dass man eine Verbindung der Formel II

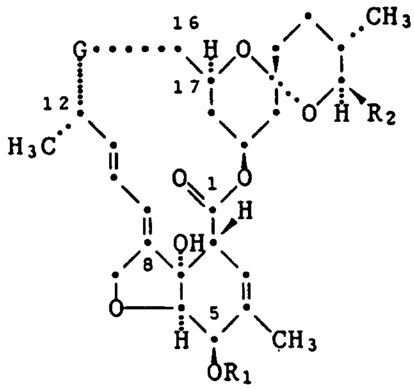

(II)

worin G für eine der Gruppen a oder b

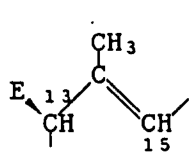

(a) oder

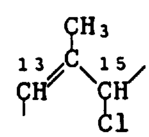

(b)

[= 13β-Halogen-$\Delta^{14,15}$] [= 15-Chlor-$\Delta^{13,14}$]

steht, $R_1$ Wasserstoff oder eine OH-Schutzgruppe bedeutet, $R_2$ die für Formel I angegebenen Bedeutungen hat und E für Chlor, Brom oder Jod steht, mit einer Diarylzink-Verbindung der Formel III

Ph-Zn-Ph (III),

worin Ph die für Formel I angegebene Bedeutung hat, in Gegenwart eines Uebergangsmetallsalzes umsetzt und gewünschtenfalls, wenn $R_1$ für eine OH-Schutzgruppe steht, diese Schutzgruppe hydrolytisch abspaltet.

**2.** Verfahren nach Anspruch 1 zur Herstellung von Verbindungen der Formel I, worin X -CH($OR_1$)- und $R_1$ Wasserstoff oder eine Schutzgruppe bedeutet, $R_2$ für Methyl, Ethyl, Isopropyl oder sek.-Butyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**3.** Verfahren nach Anspruch 1 zur Herstellung von Verbindungen der Formel I, worin X -CH($OR_1$)- und $R_1$ Wasserstoff bedeutet, $R_2$ für Methyl, Ethyl, Isopropyl oder sek.-Butyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**4.** Verfahren nach Anspruch 1 zur Herstellung von Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy, $C_2$-$C_{10}$-Alkoxyalkoxy oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**5.** Verfahren nach Anspruch 1 zur Herstellung von Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_{10}$-Alkyl, $C_2$-$C_{10}$-Alkoxyalkyl, $C_2$-$C_{10}$-Alkenyl, $C_1$-$C_{10}$-Alkoxy oder $C_2$-$C_{10}$-Alkoxyalkoxy stehen.

**6.** Verfahren nach Anspruch 1 zur Herstellung von Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff oder einen unsubstituierten oder durch mindestens einen Substituenten der Gruppe $C_1$-$C_3$-Alkyl oder $C_1$-$C_3$-Alkoxy substituierten Phenyl- oder Phenoxyrest stehen.

**7.** Verfahren nach Anspruch 1 zur Herstellung von Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, $C_1$-$C_3$-Alkyl, oder $C_1$-$C_3$-Alkoxy stehen, mit der Massgabe, dass höchstens zwei der Substituenten $R_a$, $R_b$, $R_c$ und $R_d$ eine andere Bedeutung als Wasserstoff haben.

**8.** Verfahren nach Anspruch 1 zur Herstellung von Verbindungen der Formel I, worin X -CH($OR_1$)- und $R_1$ Wasserstoff bedeutet, $R_2$ für Methyl oder Ethyl steht; und Ph einen durch $R_a$, $R_b$, $R_c$ und $R_d$ substituierten Phenylring darstellt, wobei $R_a$, $R_b$, $R_c$ und $R_d$ unabhängig voneinander für Wasserstoff, Phenyl oder Phenoxy stehen, mit der Massgabe, dass höchstens zwei der Substituenten $R_a$, $R_b$, $R_c$ und $R_d$ eine andere Bedeutung als Wasserstoff haben.

**9.** Verfahren nach Anspruch 1 zur Herstellung von Verbindungen der Formel I, worin X -CH($OR_1$)- und $R_1$ Wasserstoff bedeutet, $R_2$ für Methyl oder Ethyl, steht; und Ph für unsubstituiertes oder durch Methyl, Methoxy, Phenyl oder Phenoxy monosubstituiertes oder durch Methyl oder Methoxy disubstituiertes oder durch Methyl trisubstituiertes Phenyl steht.

**10.** Verfahren nach Anspruch 1 zur Herstellung von Verbindungen der Formel I worin X -CH($OR_1$)- und $R_1$ Wasserstoff bedeutet, $R_2$ für Ethyl steht; und Ph für unsubstituiertes oder durch Methyl, Methoxy, Phenyl oder Phenoxy monosubstituiertes oder durch Methyl oder Methoxy disubstituiertes oder durch

Methyl trisubstituiertes Phenyl steht.

11. Verfahren nach Anspruch 1 zur Herstellung von Verbindungen der Formel I, worin X -CH($OR_1$)- und $R_1$ Wasserstoff bedeutet, $R_2$ für Methyl oder insbesondere für Ethyl steht; und Ph für unsubstituiertes oder durch Methyl, Methoxy, Phenyl oder Phenoxy monosubstituiertes oder durch Methoxy disubstituiertes Phenyl steht.

12. Verfahren nach Anspruch 1 zur Herstellung einer Verbindung der Formel I ausgewählt aus der Gruppe:
13β-Phenyl-milbemycin $A_4$,
13β-(2-Methoxyphenyl)-milbemycin $A_4$,
13β-(3-Methoxyphenyl)-milbemycin $A_4$,
13β-(4-Methoxyphenyl)-milbemycin $A_4$,
13β-(2-Methylphenyl)-milbemycin $A_4$,
13β-(4-Biphenylyl)-milbemycin $A_4$,
13β-(4-Phenoxyphenyl)-milbemycin $A_4$ und
13β-(3,4-Dimethoxyphenyl)-milbemycin $A_4$.

13. Verfahren nach Anspruch 1 zur Herstellung einer Verbindung der Formel I ausgewählt aus der Gruppe:
13β-Phenyl-milbemycin $A_3$,
13β-(2-Methylphenyl)-milbemycin $A_4$ und
13β-(4-Methylphenyl)-milbemycin $A_4$.

14. Verfahren nach Anspruch 1, dadurch gekennzeichnet, dass man die Umsetzung in Gegenwart eines Uebergangsmetallsalzes, welches als metallische Komponente Fe, Ru, Os, Co, Rh, Ir, Ni, Pd oder Pt enthält, durchführt.

15. Verfahren nach Anspruch 14, dadurch gekennzeichnet, dass man die Umsetzung in Gegenwart eines Uebergangsmetallsalzes, welches als metallische Komponente Co, Ni oder Pd enthält, durchführt.

16. Verfahren nach Anspruch 1, dadurch gekennzeichnet, dass man als Uebergangsmetallsalz $NiCl_2$, $CoCl_2$, $[(Phenyl)_3P]_2NiCl_2$, $[(Phenyl)_3P[_2PdCl_2$, $[(Phenyl)_2PCH_2]_2NiCl_2$, $[(Phenyl)_2PCH_2]_2CoBr_2$, [-$(Phenyl)_2PCH_2CH_2CH_2P(Phenyl)_2]NiCl_2$, $[(Phenyl)_2PCH_2CH_2]_2NiCl_2$ oder $[(Cyclohexyl)_3P]_2NiCl_2$ verwendet.

17. Verfahren zur Herstellung von Verbindungen der Formel IIb

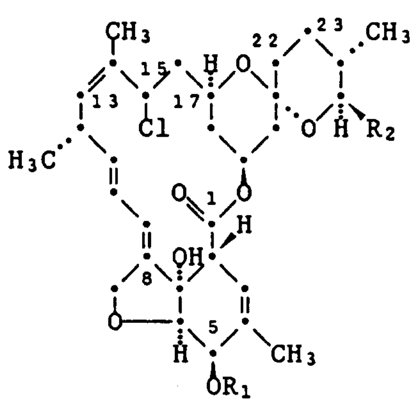

(IIb)

in denen
$R_1$ Wasserstoff oder eine OH-Schutzgruppe bedeutet; und
$R_2$ für Methyl, Ethyl, Isopropyl oder sek.Butyl steht,
dadurch gekennzeichnet, dass man ein Milbemycin der Formel M

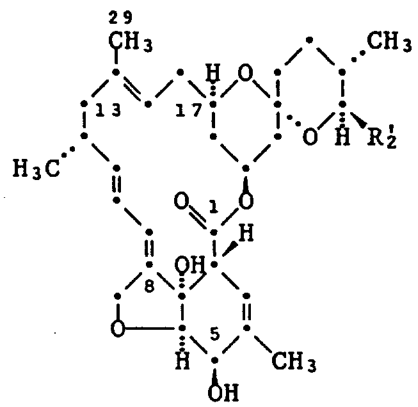

(M)

worin $R_2'$ für Methyl, Ethyl, Isopropyl oder sek.-Butyl steht, mit einem die Bildung des entsprechenden 15-Chlor-$\Delta^{13,14}$-Derivats bewirkenden Chlorierungsmittel umsetzt.

**18.** Verfahren nach Anspruch 17, dadurch gekennzeichnet, dass man als Chlorierungsmittel tert.-Butylhypochlorit verwendet.

**19.** Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I wie in einem der Ansprüche 1 bis 13 definiert, enthält.

**20.** Verfahren zur Bekämpfung von Schädlingen, dadurch gekennzeichnet, dass man eine pestizid-wirksame Menge mindestens einer Verbindung der Formel I wie in einem der Ansprüche 1 bis 13 definierte auf oder in die Wirtspflanzen oder sonstigen Lebensräume der Schädlinge mit Ausnahme menschlicher oder tierischer Körper appliziert.

**21.** Verfahren nach Anspruch 20, dadurch gekennzeichnet, dass die zu bekämpfenden Schädlinge Tiere befallende Endo- oder Ektoparasiten sind.

**22.** Verfahren nach Anspruch 20, dadurch gekennzeichnet, dass die zu bekämpfenden Schädlinge pflanzenschädigende Parasiten sind.

**23.** Verfahren nach Anspruch 20, dadurch gekennzeichnet, dass es sich bei den Schädlingen um parasitäre Nematoden handelt.

**24.** Verbindungen der Formel I wie in einem der Ansprüche 1 bis 13 definiert, zur Verwendung als antiparasitäres Mittel gegen Tiere befallende Endo- und Ektoparasiten.

**25.** Verwendung einer Verbindung der Formel I wie in einem der Ansprüche 1 bis 13 definiert, zur Herstellung eines Arzneimittels zur Bekämpfung von Tiere befallenden Endo- und Ektoparasiten.

**26.** Verfahren zur Herstellung eines Mittels wie im Anspruch 19 definiert, dadurch gekennzeichnet, dass man eine Verbindung der Formel I wie in einem der Ansprüche 1 bis 13 definiert, mit Trägermitteln, Verteilungsmitteln oder Träger- und Verteilungsmitteln vermischt, vermahlt oder vermischt und vermahlt.

## Claims

**Claims for the following Contracting States : AT, BE, CH, DE, FR, GB, IT, LI, LU, NL, SE**

**1.** A compound of formula I

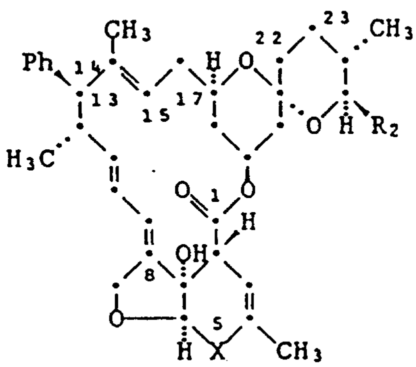

(I)

in which

X is one of the groups -CH($OR_1$)-, -C(O)- or -C(=N-OR)-;
$R_1$ is hydrogen or an OH-protecting group;
R is hydrogen, an OH-protecting group, or an alkyl or cycloalkyl group;
$R_2$ is methyl, ethyl, isopropyl or sec-butyl;
and
Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**2.** A compound of formula I according to claim 1, wherein X is -CH($OR_1$)- and $R_1$ is hydrogen or a protecting group, $R_2$ is methyl, ethyl, isopropyl or sec-butyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**3.** A compound of formula I according to claim 1, wherein X is -CH($OR_1$)- and $R_1$ is hydrogen, $R_2$ is methyl, ethyl, isopropyl or sec-butyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**4.** A compound of formula I according to claim 1, wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**5.** A compound of formula I according to claim 1, wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy or $C_2$-$C_{10}$alkoxyalkoxy.

**6.** A compound of formula I according to claim 1, wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, or a phenyl or phenoxy radical that is

unsubstituted or is substituted by at least one substituent from the group $C_1$-$C_3$alkyl and $C_1$-$C_3$alkoxy.

**7.** A compound of formula I according to claim 1, wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_3$alkyl or $C_1$-$C_3$alkoxy, with the proviso that not more than two of the substituents $R_a$, $R_b$, $R_c$ and $R_d$ have a meaning other than hydrogen.

**8.** A compound of formula I according to claim 1, wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, phenyl or phenoxy, with the proviso that not more than two of the substituents $R_a$, $R_b$, $R_c$ and $R_d$ have a meaning other than hydrogen.

**9.** A compound of formula I according to claim 1, wherein X is -CH($OR_1$)- and $R_1$ is hydrogen, $R_2$ is methyl or ethyl; and Ph is phenyl that is unsubstituted or monosubstituted by methyl, methoxy, phenyl or phenoxy, or disubstituted by methyl or methoxy, or trisubstituted by methyl.

**10.** A compound of formula I according to claim 1, wherein X is -CH($OR_1$)- and $R_1$ is hydrogen, $R_2$ is ethyl; and Ph is phenyl that is unsubstituted or monosubstituted by methyl, methoxy, phenyl or phenoxy, or disubstituted by methyl or methoxy, or trisubstituted by methyl.

**11.** A compound of formula I according to claim 1, wherein X is -CH($OR_1$)- and $R_1$ is hydrogen, $R_2$ is methyl or, especially, ethyl; and Ph is phenyl that is unsubstituted or monosubstituted by methyl, methoxy, phenyl or phenoxy, or disubstituted by methoxy.

**12.** A compound of formula I selected from the group:
13β-phenyl-milbemycin $A_4$,
13β-(2-methoxyphenyl)-milbemycin $A_4$,
13β-(3-methoxyphenyl)-milbemycin $A_4$,
13β-(4-methoxyphenyl)-milbemycin $A_4$,
13β-(2-methylphenyl)-milbemycin $A_4$,
13β-(4-biphenylyl)-milbemycin $A_4$,
13β-(4-phenoxyphenyl)-milbemycin $A_4$ and
13β-(3,4-dimethoxyphenyl)-milbemycin $A_4$.

**13.** A compound of formula I selected from the group:
13β-phenyl-milbemycin $A_3$,
13β-(2-methylphenyl)-milbemycin $A_4$ and
13β-(4-methylphenyl)-milbemycin $A_4$.

**14.** A process for the preparation of a compound of formula I, which comprises reacting a compound of formula II

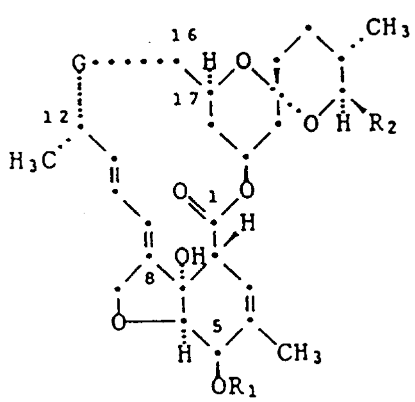

(II)

wherein G is one of the groups a or b

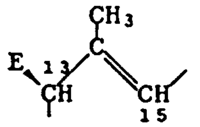 (a) or 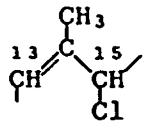 (b),

[= 13β-halogen-$\Delta^{14,15}$] [= 15-chloro-$\Delta^{13,14}$]

$R_1$ is hydrogen or an OH-protecting group, $R_2$ is as defined for formula I, and E is chlorine, bromine or iodine, with a diaryl zinc compound of formula III

Ph-Zn-Ph (III),

wherein Ph is as defined for formula I, in the presence of a transition metal salt, and, if desired, when $R_1$ is an OH-protecting group, removing that protecting group by hydrolysis.

**15.** A process according to claim 14, which comprises carrying out the reaction in the presence of a transition metal salt that comprises, as the metal component, Fe, Ru, Os, Co, Rh, Ir, Ni, Pd or Pt.

**16.** A process according to claim 15, which comprises carrying out the reaction in the presence of a transition metal salt that comprises, as the metal component, Co, Ni or Pd.

**17.** A process according to claim 14, which comprises using as the transition metal salt $NiCl_2$, $CoCl_2$, $[(phenyl)_3P]_2NiCl_2$, $[(phenyl)_3P]_2PdCl_2$, $[(phenyl)_2PCH_2]_2NiCl_2$, $[(phenyl)_2PCH_2]_2CoBr_2$, $[(phenyl)_2PCH_2CH_2CH_2P(phenyl)_2]NiCl_2$, $[(phenyl)_2PCH_2CH_2]_2NiCl_2$ or $[(cyclohexyl)_3P]_2NiCl_2$.

**18.** A process for the preparation of a compound of formula IIb

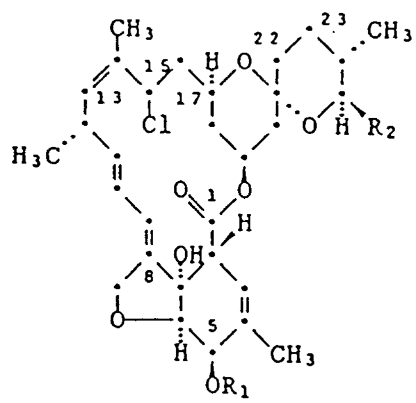 (IIb),

in which
$R_1$ is hydrogen or an OH-protecting group; and
$R_2$ is methyl, ethyl, isopropyl or sec-butyl,
which comprises reacting a milbemycin of formula M

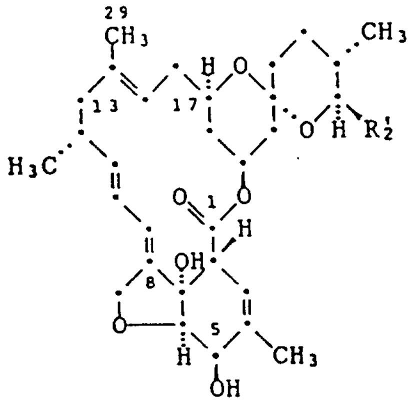

(M),

wherein $R_2'$ is methyl, ethyl, isopropyl or sec-butyl, with a chlorinating agent that brings about the formation of the corresponding 15-chloro-$\Delta^{13,14}$ derivative.

**19.** A process according to claim 18, which comprises using tert-butyl hypochlorite as the chlorinating agent.

**20.** A compound of formula IIb

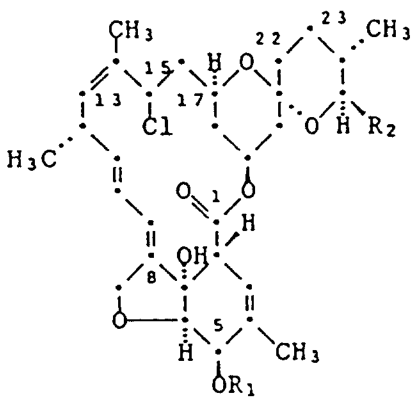

(IIb),

in which

$R_1$ is hydrogen or an OH-protecting group; and

$R_2$ is methyl, ethyl, isopropyl or sec-butyl.

**21.** A composition for controlling pests, which comprises at least one compound of formula I according to claim 1 as active ingredient, together with carriers, distributing agents or carriers and distributing agents.

**22.** A method of controlling pests, which comprises applying or administering a pesticidally effective amount of at least one compound of formula I according to claim 1 to the host plants or other loci of the pests, with the exception of the human or animal body.

**23.** A method according to claim 22, wherein the pests to be controlled are endo- or ecto-parasites of animals.

**24.** A method according to claim 22, wherein the pests to be controlled are plant-destructive parasites.

**25.** A method according to claim 22, wherein the pests are parasitic nematodes.

**26.** A compound of formula I according to claim 1 for use as an anti-parasitic agent against endo- and ecto-parasites of animals.

**27.** The use of a compound of formula I for the preparation of a medicament for controlling endo- and ecto-parasites of animals.

**Claims for the following Contracting States : ES, GR**

**1.** A process for the preparation of a compound of formula I

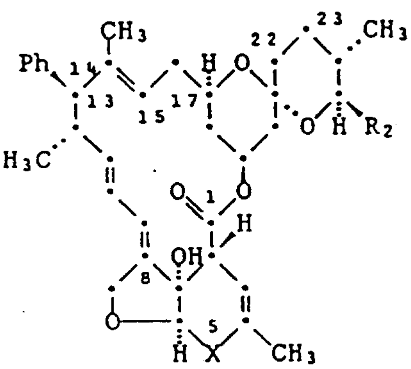

(I)

in which

| | |
|---|---|
| X | is one of the groups -CH($OR_1$)-, -C(O)- or -C(=N-OR)-; |
| $R_1$ | is hydrogen or an OH-protecting group; |
| R | is hydrogen, an OH-protecting group, or an alkyl or cycloalkyl group; |
| $R_2$ | is methyl, ethyl, isopropyl or sec-butyl; and |
| Ph | is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy, which comprises reacting a compound of formula II |

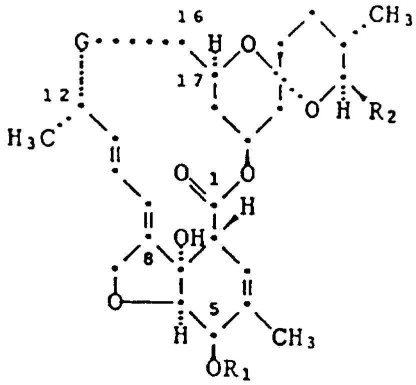

(II)

wherein G is one of the groups a or b

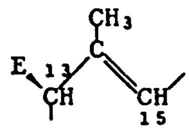 (a) or 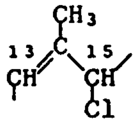 (b),

[= 13β-halogen-$\Delta^{14,15}$] [= 15-chloro-$\Delta^{13,14}$]

$R_1$ is hydrogen or an OH-protecting group, $R_2$ is as defined for formula I, and E is chlorine, bromine or iodine, with a diaryl zinc compound of formula III

Ph-Zn-Ph (III),

wherein Ph is as defined for formula I, in the presence of a transition metal salt, and, if desired, when $R_1$ is an OH-protecting group, removing that protecting group by hydrolysis.

**2.** A process according to claim 1 for the preparation of a compound of formula I wherein X is -CH($OR_1$)- and $R_1$ is hydrogen or a protecting group, $R_2$ is methyl, ethyl, isopropyl or sec-butyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$-alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**3.** A process according to claim 1 for the preparation of a compound of formula I wherein X is -CH($OR_1$)- and $R_1$ is hydrogen, $R_2$ is methyl, ethyl, isopropyl or sec-butyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**4.** A process according to claim 1 for the preparation of a compound of formula I wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy, $C_2$-$C_{10}$alkoxyalkoxy, or a phenyl or phenoxy radical that is unsubstituted or is substituted by at least one substituent from the group $C_1$-$C_3$alkyl and $C_1$-$C_3$alkoxy.

**5.** A process according to claim 1 for the preparation of a compound of formula I wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_{10}$alkyl, $C_2$-$C_{10}$alkoxyalkyl, $C_2$-$C_{10}$alkenyl, $C_1$-$C_{10}$alkoxy or $C_2$-$C_{10}$alkoxyalkoxy.

**6.** A process according to claim 1 for the preparation of a compound of formula I wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, or a phenyl or phenoxy radical that is unsubstituted or is substituted by at least one substituent from the group $C_1$-$C_3$alkyl and $C_1$-$C_3$alkoxy.

**7.** A process according to claim 1 for the preparation of a compound of formula I wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, $C_1$-$C_3$alkyl or $C_1$-$C_3$alkoxy, with the proviso that not more than two of the substituents $R_a$, $R_b$, $R_c$ and $R_d$ have a meaning other than hydrogen.

**8.** A process according to claim 1 for the preparation of a compound of formula I wherein X is -CH($OR_1$)- and $R_1$ is hydrogen, $R_2$ is methyl or ethyl; and Ph is a phenyl ring that is substituted by $R_a$, $R_b$, $R_c$ and $R_d$, wherein each of $R_a$, $R_b$, $R_c$ and $R_d$, independently of the others, is hydrogen, phenyl or phenoxy,

with the proviso that not more than two of the substituents $R_a$, $R_b$, $R_c$ and $R_d$ have a meaning other than hydrogen.

**9.** A process according to claim 1 for the preparation of a compound of formula I wherein X is $-CH(OR_1)-$ and $R_1$ is hydrogen, $R_2$ is methyl or ethyl; and Ph is phenyl that is unsubstituted or monosubstituted by methyl, methoxy, phenyl or phenoxy, or disubstituted by methyl or methoxy, or trisubstituted by methyl.

**10.** A process according to claim 1 for the preparation of a compound of formula I wherein X is $-CH(OR_1)-$ and $R_1$ is hydrogen, $R_2$ is ethyl; and Ph is phenyl that is unsubstituted or monosubstituted by methyl, methoxy, phenyl or phenoxy, or disubstituted by methyl or methoxy, or trisubstituted by methyl.

**11.** A process according to claim 1 for the preparation of a compound of formula I wherein X is $-CH(OR_1)-$ and $R_1$ is hydrogen, $R_2$ is methyl or, especially, ethyl; and Ph is phenyl that is unsubstituted or monosubstituted by methyl, methoxy, phenyl or phenoxy, or disubstituted by methoxy.

**12.** A process according to claim 1 for the preparation of a compound of formula I selected from the group:
13$\beta$-phenyl-milbemycin $A_4$,
13$\beta$-(2-methoxyphenyl)-milbemycin $A_4$,
13$\beta$-(3-methoxyphenyl)-milbemycin $A_4$,
13$\beta$-(4-methoxyphenyl)-milbemycin $A_4$,
13$\beta$-(2-methylphenyl)-milbemycin $A_4$,
13$\beta$-(4-biphenylyl)-milbemycin $A_4$,
13$\beta$-(4-phenoxyphenyl)-milbemycin $A_4$ and
13$\beta$-(3,4-dimethoxyphenyl)-milbemycin $A_4$.

**13.** A process according to claim 1 for the preparation of a compound of formula I selected from the group:
13$\beta$-phenyl-milbemycin $A_3$,
13$\beta$-(2-methylphenyl)-milbemycin $A_4$ and
13$\beta$-(4-methylphenyl)-milbemycin $A_4$.

**14.** A process according to claim 1, which comprises carrying out the reaction in the presence of a transition metal salt that comprises, as the metal component, Fe, Ru, Os, Co, Rh, Ir, Ni, Pd or Pt.

**15.** A process according to claim 14, which comprises carrying out the reaction in the presence of a transition metal salt that comprises, as the metal component, Co, Ni or Pd.

**16.** A process according to claim 1, which comprises using as the transition metal salt $NiCl_2$, $CoCl_2$, $[(phenyl)_3P]_2NiCl_2$, $[(phenyl)_3P]_2PdCl_2$, $[(phenyl)_2PCH_2]_2NiCl_2$, $[(phenyl)_2PCH_2]_2CoBr_2$, $[(phenyl)_2PCH_2CH_2CH_2P(phenyl)_2]NiCl_2$, $[(phenyl)_2PCH_2CH_2]_2NiCl_2$ or $[(cyclohexyl)_3P]_2NiCl_2$.

**17.** A process for the preparation of a compound of formula IIb

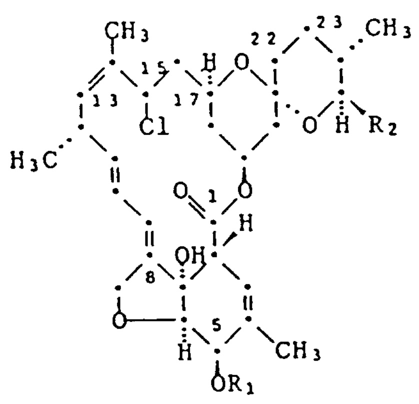

(IIb),

in which

$R_1$ is hydrogen or an OH-protecting group; and

$R_2$ is methyl, ethyl, isopropyl or sec-butyl,

which comprises reacting a milbemycin of formula M

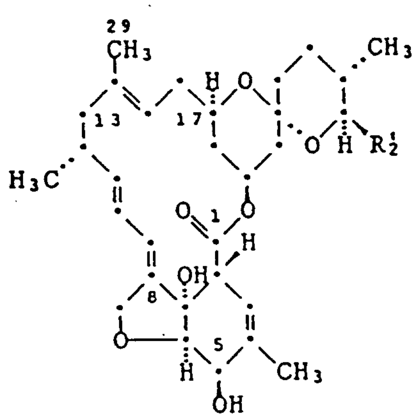

(M),

wherein $R_2'$ is methyl, ethyl, isopropyl or sec-butyl, with a chlorinating agent that brings about the formation of the corresponding 15-chloro-$\Delta^{13,14}$ derivative.

**18.** A process according to claim 17, which comprises using tert-butyl hypochlorite as the chlorinating agent.

**19.** A composition for controlling pests, which comprises as active ingredient at least one compound of formula I as defined in any one of claims 1 to 13, together with carriers, distributing agents or carriers and distributing agents.

**20.** A method of controlling pests, which comprises applying or administering a pesticidally effective amount of at least one compound of formula I as defined in any one of claims 1 to 13 to the host plants or other loci of the pests, with the exception of the human or animal body.

**21.** A method according to claim 20, wherein the pests to be controlled are endo- or ecto-parasites of animals.

**22.** A method according to claim 20, wherein the pests to be controlled are plant-destructive parasites.

**23.** A method according to claim 20, wherein the pests are parasitic nematodes.

**24.** A compound of formula I as defined in any one of claims 1 to 13 for use as an anti-parasitic agent against endo- and ecto-parasites of animals.

**25.** The use of a compound of formula I as defined in any one of claims 1 to 13 for the preparation of a medicament for controlling endo- and ecto-parasites of animals.

**26.** A process for the preparation of a composition as defined in claim 19, wherein a compound of formula I as defined in any one of claims 1 to 13 is mixed, ground or mixed and ground with carriers, distributing agents or with carriers and distributing agents.

**Revendications**

**Revendications pour les Etats contractants suivants : AT, BE, CH, DE, FR, GB, IT, LI, LU, NL, SE**

1. Composés de formule I

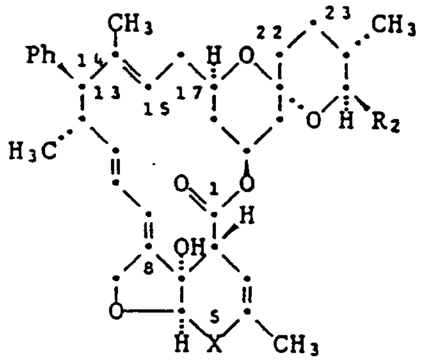

(I)

dans laquelle

X représente l'un des groupes -CH($OR_1$)-, -C(O)- ou -C(=N-OR)-;
$R_1$ représente l'hydrogène ou un groupe protecteur du groupe OH;
R représente l'hydrogène, un groupe protecteur du groupe OH, un groupe alkyle ou cycloalkyle;
$R_2$ représente un groupe méthyle, éthyle, isopropyle ou sec-butyle; et
Ph représente un noyau phényle substitué par $R_a$, $R_b$, $R_c$ et $R_d$, $R_a$, $R_b$, $R_c$ et $R_d$ représentant chacun, indépendamment les une des autres, l'hydrogène, un groupe alkyle en C1-C10, alcoxyalkyle en C2-C10, alcényle en C2-C10, alcoxy en C1-C10, alcoxyalcoxy en C2-C10, ou un groupe phényle ou phénoxy non substitué ou portant au moins un substituant choisi parmi les groupes alkyle en C1-C3 et alcoxy en C1-C3.

2. Composés de formule I selon revendication 1, dans laquelle X représente -CH($OR_1$)- et $R_1$ représente l'hydrogène ou un groupe protecteur, $R_2$ représente un groupe méthyle, éthyle, isopropyle ou sec-butyle; et Ph représente un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10, ou un groupe phényle ou phénoxy non substitué ou portant au moins un substituant choisi parmi les groupes alkyle en C1-C3 et alcoxy en C1-C3.

3. Composés de formule I selon revendication 1, dans laquelle X représente -CH($OR_1$)- et $R_1$ représente l'hydrogène, $R_2$ un groupe méthyle, éthyle, isopropyle ou sec-butyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10 ou un groupe phényle ou phénoxy non substitué ou portant au moins un substituant choisi parmi les groupes alkyle en C1-C3 et alcoxy en C1-C3.

4. Composés de formule I selon revendication 1 dans laquelle X représente -CH($OR_1$)- et $R_1$ l'hydrogène,$R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10 ou un groupe phényle ou phénoxy non substitué ou portant au moins un substituant choisi parmi les groupes alkyle en C1-C3 et alcoxy en C1-C3.

5. Composés de formule I selon revendication 1, dans laquelle X représente -CH($OR_1$)- et $R_1$ l'hydrogène,$R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10 ou alcoxyalcoxy en C2-C10.

6. Composés de formule I selon revendication 1, dans laquelle X représente -CH($OR_1$)- et $R_1$ l'hydrogène,$R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène ou un groupe phényle ou phénoxy non substitué ou portant au moins un substituant choisi parmi les groupes alkyle en C1-C3 et alcoxy en C1-C3.

7. Composés de formule I selon revendication 1, dans laquelle X représente -CH($OR_1$)- et $R_1$ l'hydrogène,$R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C3 ou alcoxy en C1-C3, sous réserve que deux des symboles $R_a$, $R_b$, $R_c$ et $R_d$ au maximum aient une signification autre que l'hydrogène.

8. Composés de formule I selon revendication 1, dans laquelle X représente -CH($OR_1$)- et $R_1$ l'hydrogène,$R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe phényle ou phénoxy, sous réserve que deux des symboles $R_a$, $R_b$, $R_c$ et $R_d$ au maximum aient une signification autre que l'hydrogène.

9. Composés de formule I selon revendication 1, dans laquelle X représente -CH($OR_1$)- et $R_1$ l'hydrogène,$R_2$ un groupe méthyle ou éthyle; et Ph un groupe phényle non substitué ou monosubstitué par un groupe méthyle, méthoxy, phényle ou phénoxy, ou disubstitué par des groupes méthyle ou méthoxy, ou trisubstitué par des groupes méthyle.

10. Composés de formule I selon revendication 1, dans laquelle X représente -CH($OR_1$)- et $R_1$ l'hydrogène, $R_2$ un groupe éthyle; et Ph un groupe phényle non substitué ou monosubstitué par un groupe méthyle, méthoxy, phényle ou phénoxy, ou disubstitué par des groupes méthyle ou méthoxy, ou trisubstitué par des groupes méthyle.

11. Composés de formule I selon revendication 1, dans laquelle X représente -CH($OR_1$)- et $R_1$ l'hydrogène, $R_2$ un groupe méthyle ou plus spécialement éthyle; et Ph un groupe phényle non substitué ou monosubstitué par un groupe méthyle, méthoxy, phényle ou phénoxy, ou disubstitué par des groupes méthoxy.

12. Un composé de formule I choisi dans le groupe suivant :
13bêta-phényl-milbémycine A4,
13bêta-(2-méthoxyphényl)-milbémycine A4,
13bêta-(3-méthoxyphényl)-milbémycine A4,
13bêta-(4-méthoxyphényl)-milbémycine A4,
13bêta-(2-méthylphényl)-milbémycine A4,
13bêta-(4-biphénylyl)-milbémycine A4,
13bêta-(4-phénoxyphényl)-milbémycine A4 et
13bêta-(3,4-diméthoxyphényl)-milbémycine A4.

13. Un composé de formule I choisi dans le groupe suivant :
13bêta-phényl-milbémycine A3,
13bêta-(2-méthylphényl)-milbémycine A4 et
13bêta-(4-méthylphényl)-milbémycine A4.

14. Procédé pour la préparation des composés de formule I, caractérisé en ce que l'on fait réagir un composé de formule II

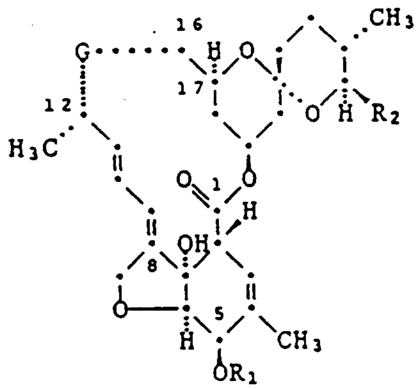

(II)

dans laquelle G représente l'un des groupes a) ou b)

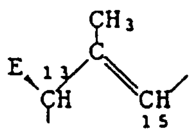 (a) ou 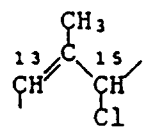

[= 13β-halogène-$\Delta^{14,15}$]   [= 15-chloro-$\Delta^{13,14}$]

$R_1$ représente l'hydrogène ou un groupe protecteur du groupe OH, $R_2$ a les significations indiquées en référence à la formule I et E représente le chlore, le brome ou l'iode, avec un diaryl-zinc de formule III

Ph-Zn-Ph   (III)

dans laquelle Ph a les significations indiquées en référence à la formule I, en présence d'un sel d'un métal de transition, après quoi, si on le désire, lorsque $R_1$ représente un groupe protecteur du groupe OH, on élimine ce groupe protecteur par hydrolyse.

**15.** Procédé selon revendication 14, caractérisé en ce que l'on effectue la réaction en présence d'un sel d'un métal de transition contenant en tant que métal composant Fe, Ru, Os, Co, Rh, Ir, Ni, Pd ou Pt.

**16.** Procédé selon revendication 15, caractérisé en ce que l'on effectue la réaction en présence d'un sel d'un métal de transition contenant en tant que métal composant Co, Ni ou Pd.

**17.** Procédé selon revendication 14, caractérisé en ce que l'on utilise en tant que sel de métal de transition $NiCl_2$, $CoCl_2$, $[(phényl)_3P]_2NiCl_2$, $[(phényl)_3P]_2PdCl_2$, $[(phényl)_2PCH_2]_2NiCl_2$, $[(phényl)_2PCH_2]_2CoBr_2$, $[-(phényl)_2PCH_2CH_2CH_2P(phényl)_2]NiCl_2$, $[(phényl)_2PCH_2CH_2]_2NiCl_2$ ou $[(cyclohexyl)_3P]_2NiCl_2$.

**18.** Procédé de préparation des composés de formule IIb

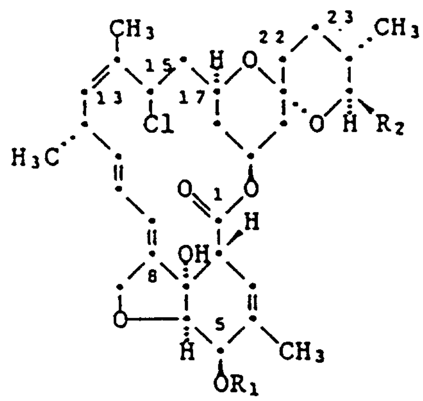

(IIb)

dans laquelle

$R_1$ représente l'hydrogène ou un groupe protecteur du groupe OH; et

$R_2$ représente un groupe méthyle, éthyle, isopropyle ou sec-butyle,

caractérisé en ce que l'on fait réagir une milbémycine de formule M,

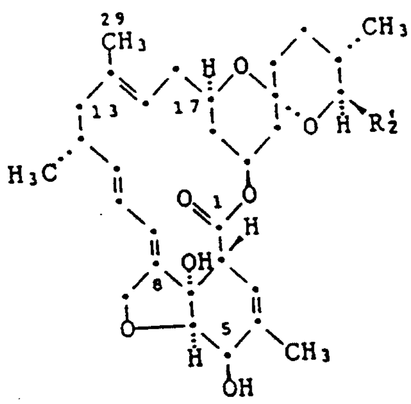

(M)

dans laquelle $R'_2$ représente un groupe méthyle, éthyle, isopropyle ou sec-butyle, avec un agent chlorant provoquant la formation du dérivé correspondant en 15-chloro-$\Delta^{13,14}$.

**19.** Procédé selon la revendication 18, caractérisé en ce que l'on utilise en tant qu'agent chlorant l'hypochlorite de tert-butyle.

**20.** Composés de formule IIb

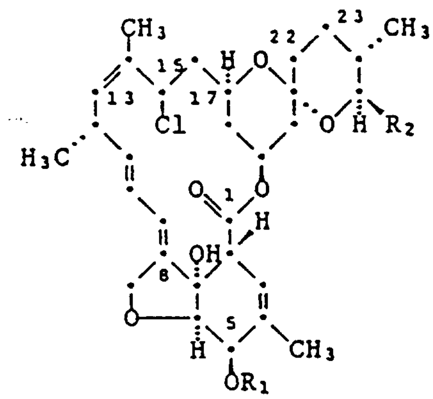

(IIb)

dans laquelle

- $R_1$ représente l'hydrogène ou un groupe protecteur du groupe OH; et
- $R_2$ représente un groupe méthyle, éthyle, isopropyle ou sec-butyle.

**21.** Produit pour combattre les parasites, caractérisé en ce qu'il contient en tant que substance active, avec des véhicules, des diluants ou des véhicules et des diluants, au moins un composé de formule I de la revendication 1.

**22.** Procédé pour combattre les parasites, caractérisé en ce que l'on applique une quantité parasiticide efficace d'au moins un composé de formule I de la revendication 1, sur ou dans les végétaux hôtes ou autres habitats des parasites, à l'exception de l'organisme humain ou animal.

**23.** Procédé selon revendication 22, caractérisé en ce que les parasites à combattre sont des endo- ou ecto-parasites des animaux.

**24.** Procédé selon revendication 22, caractérisé en ce que les parasites à combattre sont des parasites nuisibles pour les végétaux.

**25.** Procédé selon revendication 22, caractérisé en ce que les parasites sont des nématodes parasitaires.

**26.** Composés de formule I selon revendication 1, pour l'utilisation en tant qu'agents anti-parasitaires contre les endo- et ecto-parasites affectant les animaux.

**27.** Utilisation d'un composé de formule I pour la préparation d'un médicament pour combattre les endo- et ecto-parasites des animaux.

**Revendications pour les Etats contractants suivants : ES, GR**

**1.** Procédé de préparation des composés de formule I

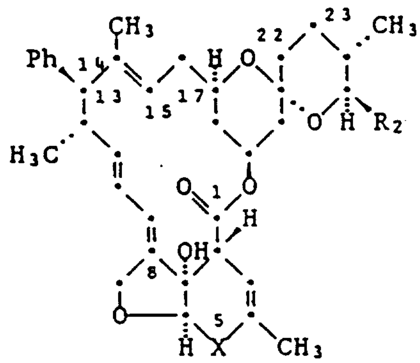

(I)

dans laquelle

- X représente l'un des groupes -CH(OR$_1$)-, -C(O)- ou - -C(=N-OR)-;
- R$_1$ représente l'hydrogène ou un groupe protecteur du groupe OH;
- R représente l'hydrogène, un groupe protecteur du groupe OH, un groupe alkyle ou cycloalkyle;
- R$_2$ représente un groupe méthyle, éthyle, isopropyle ou sec-butyle; et
- Ph représente un noyau phényle substitué par R$_a$, R$_b$, R$_c$ et R$_d$, R$_a$, R$_b$, R$_c$ et R$_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10, ou un groupe phényle ou phénoxy non substitué ou portant au moins un substituant choisi parmi les groupes alkyle en C1-C3 et alcoxy en C1-C3, caractérisé en ce que l'on fait réagir un composé de formule II

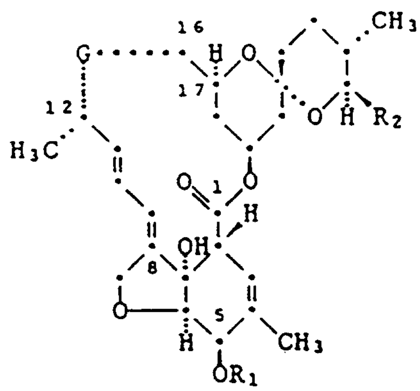

(II)

dans laquelle G représente l'un des groupes a) ou b)

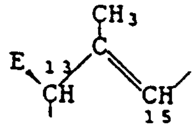

(a) ou

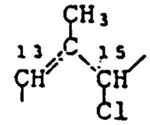

(b)

[= 13β -halogène-$\Delta^{14,15}$] [= 15-chloro-$\Delta^{13,14}$]

R$_1$ représente l'hydrogène ou un groupe protecteur du groupe OH, R$_2$ a les significations

indiquées en référence à la formule I et E représente le chlore, le brome ou l'iode, avec un diaryl-zinc de formule III

Ph-Zn-Ph (III)

dans laquelle Ph a les significations indiquées en référence à la formule I, en présence d'un sel d'un métal de transition, après quoi, si on le désire, lorsque $R_1$ représente un groupe protecteur du groupe OH, on élimine ce groupe protecteur par hydrolyse.

**2.** Procédé selon revendication 1 pour préparer les composés de formule I dans laquelle X représente -CH($OR_1$)- et $R_1$ représente l'hydrogène ou un groupe protecteur, $R_2$ représente un groupe méthyle, éthyle, isopropyle ou sec-butyle; et Ph représente un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10, ou un groupe phényle ou phénoxy non substitué ou portant au moins un substituant choisi parmi les groupes alkyle en C1-C3 et alcoxy en C1-C3.

**3.** Procédé selon revendication 1, pour la préparation des composés de formule I dans laquelle X représente -CH($OR_1$)- et $R_1$ représente l'hydrogène, $R_2$ un groupe méthyle, éthyle, isopropyle ou sec-butyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10 ou un groupe phényle ou phénoxy non substitué ou portant au moins un substituant choisi parmi les groupes alkyle en C1-C3 et alcoxy en C1-C3.

**4.** Procédé selon revendication 1, pour la préparation des composés de formule I dans laquelle X représente -CH($OR_1$)- et $R_1$ l'hydrogène, $R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10, alcoxyalcoxy en C2-C10 ou un groupe phényle ou phénoxy non substitué ou portant au moins un substituant choisi parmi les groupes alkyle en C1-C3 et alcoxy en C1-C3.

**5.** Procédé selon revendication 1, pour la préparation des composés de formule I dans laquelle X représente -CH($OR_1$)- et $R_1$ l'hydrogène, $R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C10, alcoxyalkyle en C2-C10, alcényle en C2-C10, alcoxy en C1-C10 ou alcoxyalcoxy en C2-C10.

**6.** Procédé selon revendication 1, pour la préparation des composés de formule I dans laquelle X représente -CH($OR_1$)- et $R_1$ l'hydrogène, $R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène ou un groupe phényle ou phénoxy non substitué ou portant au moins un substituant choisi parmi les groupes alkyle en C1-C3 et alcoxy en C1-C3.

**7.** Procédé selon revendication 1, pour la préparation des composés de formule I dans laquelle X représente -CH($OR_1$)- et $R_1$ l'hydrogène, $R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe alkyle en C1-C3 ou alcoxy en C1-C3, sous réserve que deux des symboles $R_a$, $R_b$, $R_c$ et $R_d$ au maximum aient une signification autre que l'hydrogène.

**8.** Procédé selon revendication 1, pour la préparation des composés de formule I dans laquelle X représente -CH($OR_1$)- et $R_1$ l'hydrogène, $R_2$ un groupe méthyle ou éthyle; et Ph un noyau phényle substitué par $R_a$, $R_b$, $R_c$ et $R_d$, $R_a$, $R_b$, $R_c$ et $R_d$ représentant chacun, indépendamment les uns des autres, l'hydrogène, un groupe phényle ou phénoxy, sous réserve que deux des symboles $R_a$, $R_b$, $R_c$ et $R_d$ au maximum aient une signification autre que l'hydrogène.

**9.** Procédé selon revendication 1, pour la préparation des composés de formule I dans laquelle X représente -CH($OR_1$)- et $R_1$ l'hydrogène, $R_2$ un groupe méthyle ou éthyle; et Ph un groupe phényle

non substitué ou monosubstitué par un groupe méthyle, méthoxy, phényle ou phénoxy, ou disubstitué par des groupes méthyle ou méthoxy, ou trisubstitué par des groupes méthyle.

**10.** Procédé selon revendication 1, pour la préparation des composés de formule I dans laquelle X représente -CH($OR_1$)- et $R_1$ l'hydrogène, $R_2$ un groupe éthyle; et Ph un groupe phényle non substitué ou monosubstitué par un groupe méthyle, méthoxy, phényle ou phénoxy, ou disubstitué par des groupes méthyle ou méthoxy, ou trisubstitué par des groupes méthyle.

**11.** Procédé selon revendication 1, pour la préparation des composés de formule I dans laquelle X représente -CH($OR_1$)- et $R_1$ l'hydrogène, $R_2$ un groupe méthyle ou plus spécialement éthyle; et Ph un groupe phényle non substitué ou monosubstitué par un groupe méthyle, méthoxy, phényle ou phénoxy, ou disubstitué par des groupes méthoxy.

**12.** Procédé selon revendication 1 pour la préparation d'un composé de formule I choisi dans le groupe suivant :
13bêta-phényl-milbémycine A4,
13bêta-(2-méthoxyphényl)-milbémycine A4,
13bêta-(3-méthoxyphényl)-milbémycine A4,
13bêta-(4-méthoxyphényl)-milbémycine A4,
13bêta-(2-méthylphényl)-milbémycine A4,
13bêta-(4-biphénylyl)-milbémycine A4,
13bêta-(4-phénoxyphényl)-milbémycine A4 et
13bêta-(3,4-diméthoxyphényl)-milbémycine A4.

**13.** Procédé selon revendication 1, pour la préparation d'un composé de formule I choisi dans le groupe suivant :
13bêta-phényl-milbémycine A3,
13bêta-(2-méthylphényl)-milbémycine A4 et
13bêta-(4-méthylphényl)-milbémycine A4.

**14.** Procédé selon revendication 1, caractérisé en ce que l'on effectue la réaction en présence d'un sel d'un métal de transition contenant en tant que métal composant Fe, Ru, Os, Co, Rh, Ir, Ni, Pd ou Pt.

**15.** Procédé selon revendication 14, caractérisé en ce que l'on effectue la réaction en présence d'un sel d'un métal de transition contenant en tant que métal composant Co, Ni ou Pd.

**16.** Procédé selon revendication 1, caractérisé en ce que l'on utilise en tant que sel de métal de transition $NiCl_2$, $CoCl_2$ $[(phényl)_3P]_2NiCl_2$, $[(phényl)_3P]_2PdCl_2$, $[(phényl)_2PCH_2]_2NiCl_2$, $[(phényl)_2PCH_2]_2CoBr_2$, [-(phényl$_2$PCH$_2$CH$_2$CH$_2$P(phényl)$_2$]NiCl$_2$, $[(phényl)_2PCH_2]_2NiCl_2$ ou $[(cyclohexyl)_3P]_2NiCl_2$.

**17.** Procédé de préparation des composés de formule IIb

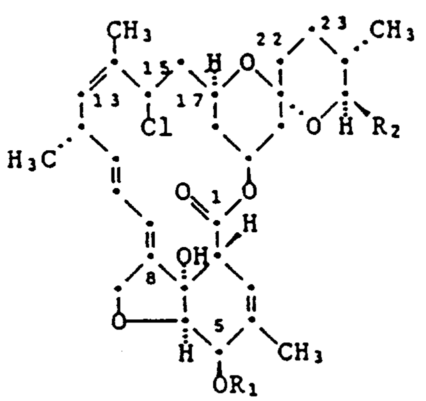

(IIb)

dans laquelle

$R_1$ représente l'hydrogène ou un groupe protecteur du groupe OH; et
$R_2$ représente un groupe méthyle, éthyle, isopropyle ou sec-butyle,
caractérisé en ce que l'on fait réagir une milbémycine de formule M

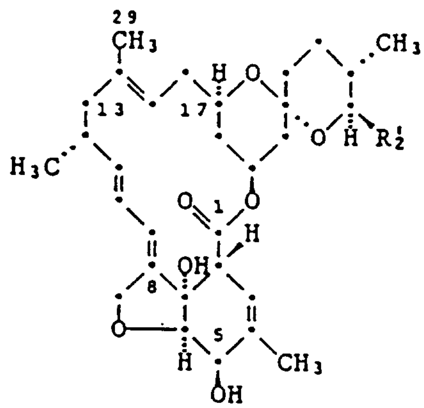

(M)

dans laquelle $R'_2$ représente un groupe méthyle, éthyle, isopropyle ou sec-butyle, avec un agent chlorant provoquant la formation du dérivé correspondant en 15-chloro-$\Delta^{13,14}$.

**18.** Procédé selon revendication 17, caractérisé en ce que l'on utilise en tant qu'agent chlorant l'hypochlorite de tert-butyle.

**19.** Produit pour combattre les parasites, caractérisé en ce qu'il contient en tant que substance active, avec des véhicules, des diluants ou des véhicules et des diluants, au moins un composé de formule I tel que défini dans l'une des revendications 1 à 13.

**20.** Procédé pour combattre les parasites, caractérisé en ce que l'on applique sur les plantes hôtes ou autres habitats des parasites, l'exception de l'organisme humain ou animal, une quantité parasiticide efficace d'au moins un composé de formule I tel que défini dans l'une des revendications 1 à 13.

**21.** Procédé selon revendication 20, caractérisé en ce que les parasites à combattre sont des endo- ou ecto-parasites des animaux.

**22.** Procédé selon revendication 20, caractérisé en ce que les parasites à combattre sont des parasites des végétaux.

**23.** Procédé selon revendication 20, caractérisé en ce que les parasites sont des nématodes parasitaires.

**24.** Composés de formule I tels que définis dans l'une des revendications 1 à 13, pour l'utilisation en tant qu'agents anti-parasitaires contre les endo- et ecto-parasites des animaux.

**25.** Utilisation d'un composé de formule I tel que défini dans l'une des revendications 1 à 13 pour la préparation d'un médicament pour la lutte contre les endo- et ecto-parasites des animaux.

**26.** Procédé de préparation d'un produit selon revendication 19, caractérisé en ce que l'on mélange, on broie ou bien on mélange et on broie un composé de formule I tel que défini dans l'une des revendications 1 à 13, avec des véhicules, des diluants ou des véhicules et des diluants.